(19) **Europäisches Patentamt / European Patent Office / Office européen des brevets**

(11) **EP 4 262 859 B1**

(12) ## EUROPEAN PATENT SPECIFICATION

(45) Date of publication and mention of the grant of the patent:
**29.01.2025 Bulletin 2025/05**

(21) Application number: **21854946.7**

(22) Date of filing: **15.12.2021**

(51) International Patent Classification (IPC):
**A61P 43/00** (2006.01)   **A61K 39/00** (2006.01)

(52) Cooperative Patent Classification (CPC):
**A61P 43/00; G16H 10/40; G16H 20/10;**
A61K 39/00; G16H 20/17

(86) International application number:
**PCT/US2021/063641**

(87) International publication number:
**WO 2022/132982 (23.06.2022 Gazette 2022/25)**

(54) **METHODS OF MANUFACTURING BIOLOGICAL THERAPIES**

VERFAHREN ZUR HERSTELLUNG BIOLOGISCHER THERAPIEN

PROCÉDÉS DE FABRICATION DE THÉRAPIES BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **16.12.2020   US 202063126274 P**
        **09.09.2021   US 202163242395 P**

(43) Date of publication of application:
**25.10.2023  Bulletin 2023/43**

(73) Proprietor: **Amgen Inc.**
**Thousand Oaks, California 91320 (US)**

(72) Inventors:
• **JOH, Nathan**
  **Thousand Oaks, CA 91320-1799 (US)**
• **JOUBERT, Marisa K.**
  **Thousand Oaks,  CA 91320-1799 (US)**
• **KLEEMANN, Gerd Richard**
  **Thousand Oaks,  CA 91320-1799 (US)**
• **TOKUDA, Joshua M.**
  **Thousand Oaks,  CA 91320-1799 (US)**
• **RIEDER, Noel J.**
  **Thousand Oaks,  CA 91320-1799 (US)**
• **NARHI, Linda Owers**
  **Thousand Oaks,  CA 91320-1799 (US)**
• **ZHANG, Zhongqi**
  **Thousand Oaks,  CA 91320-1799 (US)**
• **ZHANG, Yunlong**
  **Thousand Oaks,  CA 91320-1799 (US)**

(74) Representative: **Grünecker Patent- und Rechtsanwälte**
**PartG mbB**
**Leopoldstraße 4**
**80802 München (DE)**

(56) References cited:
• **GANDHI ARIJIT ET AL: "Quality by Design (QbD) in Pharmaceutical Industry: Tools, Perspectives and Challenges", 31 December 2016 (2016-12-31), XP055911331, Retrieved from the Internet <URL:https://www.pharmatutor. org/pdf_download/pdf/Vol.%204,%20Issue% 2011,%20November%202016,%20PharmaTutor, %20Paper-1.pdf> [retrieved on 20220411]**
• **AWOTWE-OTOO DAVID ET AL: "Quality by design: Impact of formulation variables and their interactions on quality attributes of a lyophilized monoclonal antibody", INTERNATIONAL JOURNAL OF PHARMACEUTICS, vol. 438, no. 1-2, 1 November 2012 (2012-11-01), NL, pages 167 - 175, XP055911462, ISSN: 0378-5173, DOI: 10.1016/j.ijpharm.2012.08.033**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 262 859 B1

**(Cont. next page)**

- WURTH CHRISTINE ET AL: "Quality by Design Approaches to Formulation Robustness-An Antibody Case Study", JOURNAL OF PHARMACEUTICAL SCIENCES, vol. 105, no. 5, 1 May 2016 (2016-05-01), US, pages 1667 - 1675, XP055911464, ISSN: 0022-3549, Retrieved from the Internet <URL:https://asset-pdf.scinapse.io/prod/2299618484/2299618484.pdf> DOI: 10.1016/j.xphs.2016.02.013
- ALT NADJA ET AL: "Determination of critical quality attributes for monoclonal antibodies using quality by design principles", BIOLOGICALS, ACADEMIC PRESS LTD., LONDON, GB, vol. 44, no. 5, 25 July 2016 (2016-07-25), pages 291 - 305, XP029718208, ISSN: 1045-1056, DOI: 10.1016/J.BIOLOGICALS.2016.06.005
- KEPERT J FELIX ET AL: "Establishing a control system using QbD principles", BIOLOGICALS, ACADEMIC PRESS LTD., LONDON, GB, vol. 44, no. 5, 16 July 2016 (2016-07-16), pages 319 - 331, XP029718206, ISSN: 1045-1056, DOI: 10.1016/J.BIOLOGICALS.2016.06.003
- ANDREW M. GOETZE ET AL: "Assessing monoclonal antibody product quality attribute criticality through clinical studies", MABS, vol. 2, no. 5, 1 September 2010 (2010-09-01), US, pages 500 - 507, XP055317650, ISSN: 1942-0862, DOI: 10.4161/mabs.2.5.12897
- PAUL FAYA ET AL: "Using accelerated drug stability results to inform long-term studies in shelf life determination : Informing long-term stability studies with accelerated results", STATISTICS IN MEDICINE, vol. 37, no. 17, 30 July 2018 (2018-07-30), US, pages 2599 - 2615, XP055666622, ISSN: 0277-6715, DOI: 10.1002/sim.7663

**Description**

**CROSS-REFERENCE TO RELATED APPLICATIONS**

This application claims the benefit of U.S. Provisional Application No. 63/126,274, filed December 16, 2020, entitled "Methods of Manufacturing Biological Therapies," and U.S. Provisional Application No. 63/242,395, filed September 9, 2021, entitled "Methods of Manufacturing Biological Therapies.

**FIELD**

Embodiments herein relate to methods of manufacturing biological therapies and levels of molecular attributes on biological therapies.

**BACKGROUND**

The native structure or chemistry of biological molecules (such as therapeutic proteins) adapts or alters in response to changes within the molecules' environment. Other biological therapies, including nucleic acid and cell-based therapies can also undergo changes within their environment. Although this flexibility in structure or chemistry is required for the biological function of most, if not all, biological molecules and cells, it also presents many challenges during the development and manufacture of biological therapies for pharmaceutical applications. For example, therapeutic proteins endure various conditions during the many process steps that lead up to being administered to a patient. The many process steps include, for instance, one or more of protein production (e.g., recombinant production), harvest, purification, formulation, filling, packaging, storage, delivery, and final preparation immediately prior to administration to the patient. During each of these steps, a therapeutic protein is placed in one or more environments that may or may not lead to a change in its structure or chemistry. The change in structure or chemistry can lead to the formation of different species of the biological therapies that results in a heterogeneous product. While some species retain their ability to bind to their targets and therefore maintain therapeutic efficacy, others lose target binding ability and thus become functionally inactive. In order to maximize and maintain quality control of these biological therapies, the biopharmaceutical industry has focused much effort to understand why some species lose activity while others retain activity

Molecular attributes define the physicochemical property of therapeutic biological molecules, and can therefore impact the drug safety and efficacy. The levels of attributes critical to the drug quality, or critical quality attributes (CQAs), are explicitly defined by the product purity specifications subject to extensive regulatory reviews.

**SUMMARY**

In accordance with some embodiments, a method of manufacturing a biological therapy is described. The method can comprise detecting a level of a molecular attribute of the biological therapy in a formulation at one or more timepoints under storage conditions. The method can comprise determining a rate of change of the molecular attribute under the storage conditions. The method can comprise obtaining data on *in vivo* safety and/or efficacy of the biological therapy for subjects that have received an administration of the biological therapy. The method can comprise estimating a level of molecular attribute exposure received by the subjects at the time of the administration of the biological therapy, based on (i) the rate of change of the molecular attribute during storage of the biological therapy in the formulation and (ii) a duration that the biological therapy in the formulation was under the storage conditions prior to the administration of the biological therapy. The method can comprise determining a presence or absence of a correlation between the estimated level of molecular attribute exposure and the safety and/or efficacy data for the biological therapy. If the correlation is absent, the method can comprise manufacturing production lots of the biological therapy comprising the molecular attribute at or below a specified permissible level of the molecular attribute based on the estimated level of molecule attribute exposure. If the correlation is present, the method can further comprise setting a specification for levels of the molecular attribute at the time of manufacture that do not exceed a maximum permissible level of the molecular attribute of the biological therapy. The maximum permissible level of the molecular attribute may be based on the highest estimated levels of molecular attribute exposure that are not associated with adverse events and/or inhibition of efficacy of the biological therapy. The manufacturing may further comprise rejecting production lots of the biological therapy comprising levels of the molecular attribute exceeding the maximum permissible levels. In some instances, the specified maximum permissible level of the molecular attribute is calculated to produce a level of attribute exposure at end-of-shelf that is less than or equal to 90-100% of the highest estimated level of molecular attribute exposure that is not associated with adverse events and/or an inhibition of efficacy in subjects.

In In accordance with some embodiments, a method of developing a manufacturing process for a biological therapy. The method can comprise detecting a level of a molecular attribute of the biological therapy in a formulation at one or more timepoints under storage conditions. The method can comprise determining a rate of change of the molecular attribute under the storage conditions. The method can comprise obtaining data on safety and/or efficacy of the biological therapy in subjects that have received an administration of the biological therapy. The method can comprise estimating a level of molecular attribute exposure received by the subjects at the time of the administration of the biological therapy, based on (i) the rate of change of the molecular attribute during storage of the biological therapy in the formulation and (ii) a duration that the biological therapy in the formulation was under the storage conditions prior to the administration of the biological therapy. The method can comprise determining a presence or absence of a correlation between the estimated level of molecular attribute exposure and the safety and/or efficacy data of the biological therapy. If (a) the correlation is absent, the method can comprise establishing the manufacturing process to produce levels of the molecular attribute at or below a specified permissible level based on the estimated level of molecular attribute exposure. Or, if (b) the correlation is present, the method can comprise establishing the manufacturing process to produce levels of the molecular attribute at or below a specified a maximum permissible level of the molecular attribute based on the highest level of the molecular attribute that is not associated with adverse events and/or inhibition of efficacy of the biological therapy.

In accordance with some embodiments, a method of assessing a clinical impact of a molecular attribute of a biological therapy is described. The method can comprise detecting a level of a molecular attribute of the biological therapy in a formulation at one or more timepoints under storage conditions. The method can comprise determining a rate of change of the molecular attribute under the storage conditions. The method can comprise obtaining data on safety and/or efficacy of the biological therapy in subjects that have received an administration of the biological therapy. The method can comprise estimating a level of molecular attribute exposure received by the subjects at the time of the administration of the biological therapy, based on (i) the rate of change of the molecular attribute during storage of the biological therapy in the formulation and (ii) a duration that the biological therapy in the formulation was under the storage conditions prior to said administration. The method can comprise determining a presence or absence of a correlation between the estimated molecular attribute exposure and the safety and/or efficacy of the biological therapy. If (a) the correlation is absent, the method can comprise determining that the molecular attribute impacts neither clinical safety nor efficacy of the biological therapy. Or, if (b) the correlation is present, the method can comprise determining that the molecular attribute impacts safety and/or efficacy of the biological therapy. In some instances, if (a) the correlation is absent, the method can further comprise setting a specification for permissible levels of the molecular attribute of the biological therapy, wherein the permissible levels of the molecular attribute are based on the highest estimated levels of molecular attribute exposure received by the subjects. Or, if (b) the correlation is present, the method can further comprise setting a specification for maximum permissible levels of the molecular attribute of the biological therapy, in which the maximum permissible levels of the molecular attribute are based on levels of the molecular attribute associated with adverse events and/or inhibition of efficacy of the biological therapy.

For any of the methods described herein, the estimating may be further based on (iii) a dose of the biological therapy in said administration, and (iv) an amount of the molecular attribute measured at time of manufacture and/or lot release.

For any of the methods described herein, the level of the molecular attribute of the biological therapy in the formulation may be detected at two or more timepoints under storage conditions.

For any of the methods described herein, the one or more timepoints or two or more timepoints may comprise a time of manufacture, and at least two subsequent timepoints.

For any of the methods described herein, estimating the level of the molecular attribute exposure may comprise the calculation:

$$A_t = \left\{ \%A_0 + \sum_{i=1}^{n} \left( \%A_{\Delta_i} \times t_i \right) \right\} \times D$$

in which $A_t$ is the estimated level of molecular attribute exposure, $\%A_0$ is the percentage of the molecular attribute at time of lot release, $\%A_{\Delta i}$ is the rate of change of the percentage of the molecular attribute level over time at a given storage condition, $t_i$ is the time of storage at the given condition, and $D$ is a dose strength of the administration.

For any of the methods described herein, estimating the level of the molecular attribute exposure may comprise the calculation:

$$\%A_{\text{rel.}} = \frac{A_t}{D} \times 100\%$$

,

in which $\%A_{\text{rel.}}$ is percentage of relative level of the molecular attribute exposure with respect to dosage, and in which $A_t$ is

level of the molecular attribute exposure computed using the equation 1 or 2, and in which *D* is dose strength in the dimension of weigh of active pharmaceutical ingredient associated with each treatment.

For any of the methods described herein, the correlation may comprise a weighted correlation between attribute exposure and adverse event occurrences.

For any of the methods described herein, determining a presence or absence of a correlation between the estimated level of molecular attribute exposure and the safety and/or efficacy data for the biological therapy may comprise Bayesian estimation.

For any of the methods described herein, the biological therapy in the formulation may have been under the storage conditions for different durations in the administrations to different subjects.

For any of the methods described herein, the administration (of the biological therapy) may comprise two or more administration events. In some instances, the estimated level of molecular attribute exposure received by the subjects at the time of the administration (of the biological therapy) can be amaximum or an average of the two or more administration events.

For any of the methods described herein, the administration (of the biological therapy) may comprises a continuous infusion, and the estimating may comprise calculating an estimated level of molecular attribute exposure during two or more intervals of the continuous infusion, such 24-hour intervals.

For any of the methods described herein, the safety data may comprise adverse event data. In some instances, the safety data can comprise an adverse event time course.

For any of the methods described herein, the efficacy data may comprise clinical endpoint data.

For any of the methods described herein, the molecular attribute may comprise at least one of: acidic species, basic species, high molecular weight species, subvisible particle number, low molecular weight, middle molecular weight, glycosylation (such as non-glycosylated heavy chain or high mannose), non-heavy chain and light chain, deamidation, deamination, cyclization, oxidation, isomerization, fragmentation/clipping, N-terminal and C-terminal variants, reduced and partial species, folded structure, surface hydrophobicity, chemical modification, covalent bond, a C-terminal amino acid motif PARG, or a C-terminal amino acid motif PAR-Amide. For any of the methods described herein, the molecular attribute may comprise at least one of: acidic species, basic species, high molecular weight species, amino acid isomers, or subvisible particle number.

For any of the methods described herein, the biological therapy may be selected from the group consisting of: an antibody, an antigen-binding antibody fragment, an antibody protein product, a Bi-specific T cell engager (BiTE®) molecule, a bispecific antibody, a trispecific antibody, an Fc fusion protein, a recombinant protein, a recombinant virus, a recombinant T cell, a synthetic peptide, and an active fragment of a recombinant protein.

For any of the methods described herein, the formulation may be a pharmaceutically acceptable formulation. For any of the methods described herein, the subject (or patient) may be a human subject (or patient).

For any of the methods described herein, detecting the level of a molecular attribute of the biological therapy may comprise mass spectrometry, chromatography, electrophoresis, spectroscopy, light obscuration, a particle method (such as nanoparticle/visible/micron-sized resonant mass or Brownian motion), analytical centrifugation, imaging or imaging characterization, or immunoassay.

## BRIEF DESCRIPTION OF THE DRAWINGS

FIGs. 1A-C are a series of graphs illustrating components of computational and statistical approaches for determining Clinical Impact of Attributes (CIA) in accordance with embodiments herein.

FIGs. 2A-B are a series of graphs illustrating the impact on ADA by attributes of mAb A **(FIG. 1A)** and mAb B **(FIG. 1B)** in accordance with embodiments herein. In each graph of **FIG. 2B,** results for ADA-negative patients are shown on the left, and results for ADA-positive patients are shown on the right.

FIGs. 3A-B are a series of graphs illustrating CIA analysis (subjects grouped according to the representative $A_t$) of ADA Incidence for Attributes of mAb A **(FIG. 3A)** and mAb B **(FIG. 3B)** in accordance with embodiments herein.

FIGs. 4A-B are a series of graphs illustrating CIA analysis (subjects grouped according to the representative $A_t$) of ADA Time Course for attributes of mAb A **(FIG. 4A)** and mAb B **(FIG. 4B)** in accordance with embodiments herein.

FIGs. 5A-C is a series of graphs illustrating association between ADA Response and Representative $A_t$ (amount of attribute exposure at the time of treatment administration) for mAb B. In each graph of **FIG. 5A,** results for all treatments for ADA-positive patients are shown on the left, results for all treatments for ADA-negative patients are shown in the center, and results for all treatments for ADA-relevant treatments for ADA-positive patients are shown on the right.

**FIG. 6** is a series of graphs illustrating the impact of factors in treatments on ADA. In each graph of **FIG. 6,** results for ADA-negative patients are shown on the left, and results for ADA-positive patients are shown on the right.

**FIG. 7A-B** is a series of graphs illustrating CIA analyses subjects grouped by the retrospectively known clinical outcome) (FIG. 7A) and, for subjects grouped according to the representative At, Incidence for Impact on ADA by Attributes of mAb A **(FIG. 7B).** In each graph of **FIG. 7A,** results for ADA-negative patients are shown on the left, and results for ADA-positive patients are shown on the right.

**FIG. 8** is a graph illustrating CIA analysis of subjects grouped by the retrospectively known clinical outcome for Impact on ADA by Attributes of mAb A using Advanced-Stage Clinical Studies.

**FIG. 9** is a graph illustrating Average or Maximum $A_t$ used as Representative $A_t$ Analyzed in subjects grouped according to the representative $A_t$, for Impact on ADA by Attributes of mAb B.

**FIGs. 10A-B** are graphs showing example assessments of impact of molecular attributes and potency of Product C, a canonical BiTE® molecule, on 6 adverse events. In each of the graphs of **FIG. 10A,** for each X-axis value category (0, >0-15, or >15), patients with no adverse events are shown on the left and patients with adverse events are shown on the right. In each of the graphs of **FIG. 10B,** for each X-axis value category (0, >0-15, or >15), patients with no or $G \leq 2$ adverse events are shown on the left and patients with $G \geq 3$ adverse events are shown on the right.

**FIGs. 11A-D** is a series of graphs showing an analysis of an impact of high molecular weight (HMW) species on Pyrexia using a method as described herein that does not utilize Bayesian estimation **(FIG. 11A-B)** and a method as described herein that utilizes Bayesian estimation **(FIG. 11C-D).**

## DETAILED DESCRIPTION

Described herein are methods of manufacturing a biological therapy (such as a therapeutic protein, nucleic acid, or therapy comprising cells) in which safety and efficacy of the biological therapy are controlled by limiting levels of attribute exposure to a subject. After a production lot of a biological therapy is manufactured, the biological therapy will be stored in formulation for some period of time before it is administered to a subject. During that time in storage, levels of molecular attributes of the biological therapy may change. Additionally, levels of molecular attributes may vary between different productions lots, for example reflecting differences in molecular attribute levels at different stages of production between initial cell culture and final drug product of the biological therapy. For example, levels of attributes such as acidic species, basic species, high molecular weight species, amino acid isomers, or subvisible particles may increase. Such attributes may cause a decrease in efficacy of the biological therapy, and/or may cause adverse events in a subject receiving the biological therapy. As described herein, changes in levels of molecular attributes in storage can be modeled. Based on the level of molecular attribute at the time a production lot of a biological therapy is manufactured, the amount of time that the biological therapy in formulation is in storage before administration to a subject, the rate of change of the molecular attribute in storage, and the dosage of the biological therapy, the level of molecular attribute at the time of administration to the subject can be calculated. Furthermore, if the actual level of molecular attribute at the time of administration is not associated with adverse events or a loss of efficacy, it can be determined that that level of molecular attributes is safe and effective. Accordingly, using methods described herein, production lots of the biological therapy can be produced at or below specified levels based on the level that is deemed to be safe and effective at the time of administration.

It is contemplated that while criticality and manufacturing specifications of molecular attributes have been determined using conventional methods, conventionally determined criticality and specifications for molecular attributes can present little clinical relevance. The criticality is generally investigated using non-human model systems, and the specification limits reflect the low levels of attributes reasonably achievable upon manufacture and storage. Alternatively, in a prior-knowledge or clinical-experience approach, an attribute is proposed to be not critical when clinical consequences are rarely observed for other drug products containing the attribute. This approach, however, ignores the possible product-specific variability in attribute impact. Also, adverse events can be caused by an attribute and still appear rare, if the lots that contain a high enough level of the attribute to cause the events are infrequently distributed in clinics due to lot-to-lot variability.

Methods described herein may utilize a data analysis approach that tests whether a correlation exists between the estimated actual level of patient exposure to a given attribute and the extent of the occurrence of a clinical outcome by analyzing the data from clinical studies and product quality analysis studies. This approach may be referred to as Clinical Impact of Attributes (CIA). The methods described herein utilize actual clinical data and assessments of levels of attribute exposure at the time that a biological therapy is administered. These methods provide a real-world assessment of the impact of molecular attributes, overcoming shortcomings of conventional approaches that have utilized non-human model systems for determining attribute levels, and which have not accounted for attribute exposure at the time of administration.

## Molecular attributes

"Molecular attributes" and variations of this root term has its ordinary and customary meaning as would be understood by one of ordinary skill in the art in view of this disclosure. It refers to a chemically or physically changed structure on a macromolecule, such as a protein or nucleic acid, and may be characterized in terms of its physicochemical identity or attribute type and location within the sequence of the macromolecule, e.g., the position of the amino acid on which the attribute is present. For example, asparagine and glutamine residues are susceptible to deamidation. A deamidated asparagine at position 10 of a therapeutic protein amino acid sequence is an example of an attribute. Exemplary molecular attribute types are described herein. For conciseness, molecular attributes may be referred to herein simply as "attributes." The levels of attributes critical to the drug quality, or critical quality attributes (CQAs), may be explicitly defined by the product purity specifications. These specifications are typically subject to extensive regulatory reviews. In some embodiments, a specification may set the permissible levels of one or more molecular attributes in the manufacture of a biological therapy.

In some embodiments, the molecular attribute comprises or consists of one or more of acidic species, basic species, high molecular weight species, subvisible particle number, low molecular weight, middle molecular weight, glycosylation (such as non-glycosylated heavy chain or high mannose), non-heavy chain and light chain, deamidation, deamination, cyclization, oxidation, isomerization, fragmentation/clipping, N-terminal and C-terminal variants, reduced and partial species, folded structure, surface hydrophobicity, chemical modification, covalent bonds a C-terminal amino acid motif PARG, or a C-terminal amino acid motif PAR-Amide.

PARG is an alternative C-terminal variant of antibodies that may occur as a result of alternative splicing. It represents 4 amino acids (Proline, Alanine, Arginine, Glycine) where the "AR" was genetically inserted in the canonical IgG2 C-terminal sequence. PAR-amide is another C-terminal variant that results from further processing of PARG. It refers to the C-terminal glycine getting cleaved off of antibodies ending in PARG, leaving behind an amide group on the C-terminal Arginine.

In some embodiments, the molecular attribute comprises or consists of at least one of: acidic species, basic species, high molecular weight species, amino acid isomers, or subvisible particle number.

## Techniques for detecting levels of molecular attributes

Any suitable analytical technique for detecting a molecular attribute may be used with the methods described herein. Techniques for detecting a molecular attribute include, but are not limited to mass spectrometry, chromatography, electrophoresis, spectroscopy, light obscuration, particle methods (nanoparticle/visible/micron-sized resonant mass or Brownian motion), analytical centrifugation, imaging and imaging characterizations, and immunoassays.

Example techniques for detecting molecular attributes include reduced and non-reduced peptide mapping (which may detect chemical modifications), chromatography (such as size exclusion chromatography (SEC), ion exchange chromatography (IEX) such as cation exchange chromatography (CEX), hydrophobic interaction chromatography (HIC), affinity chromatography such as Protein A-column chromatography, or reverse phase (RP) chromato-graphy), capillary isoelectric focusing (cIEF), capillary zone electrophoresis (CZE), free flow fractionation (FFF), or ultracentrifugation (UC), HIAC (such as for detecting subvisible particle count), MFI (such as for detecting subvisible particle count and morphology), visible inspection (visible particles), SDS-PAGE (such as for detecting fragments, covalent aggregates), color analysis (Trp Ox), rCE-SDS and nrCE-SDS (such as for detecting fragments that are partial molecules), nanoparticle sizing methods, spectroscopy methods (such as FTIR, CD, intrinsic fluorescence, or ANS dye binding), an Ellman's assay (free sulfhydryl's), SEC-MALS, HILIC (glycan map), and ELISA (such as for detecting HCP).

## Biological Therapies

As used herein "biological therapy" and variations of this root term has its ordinary and customary meaning as would be understood by one of ordinary skill in the art in view of this disclosure. It refers to a therapeutic composition comprising a biological macromolecule, for example a gene therapy, a therapeutic protein, a nucleic acid, a virus, or a cell or a portion thereof.

In methods described herein, the biological therapy may be selected from the group consisting of: an antibody, an antigen-binding antibody fragment, an antibody protein product, a Bi-specific T cell engager (BiTE®) molecule, a bispecific antibody, a trispecific antibody, an Fc fusion protein, a recombinant protein, a recombinant virus, a recombinant T cell, a synthetic peptide, deoxyribonucleic acid (DNA), ribonucleic acid (RNA), and an active fragment of a recombinant protein.

An "antibody" has its customary and ordinary meaning as understood by one of ordinary skill in the art in view of this disclosure. It refers to an immunoglobulin of any isotype with specific binding to the target antigen, and includes, for instance, chimeric, humanized, and fully human antibodies. By way of example, the antibody may be a monoclonal antibody. By way of example, human antibodies can be of any isotype, including IgG (including IgG1, IgG2, IgG3 and IgG4 subtypes), IgA (including IgA1 and IgA2 subtypes), IgM and IgE. A human IgG antibody generally will comprise two full-length heavy chains and two full-length light chains. Antibodies may be derived solely from a single source, or may be "chimeric," that is, different portions of the antibody may be derived from two or more different antibodies from the same or different species. It will be understood that once an antibody is obtained from a source, it may undergo further engineering, for example to enhance stability and folding. Accordingly, it will be understood that a "human" antibody may be obtained from a source, and may undergo further engineering, for example in the Fc region. The engineered antibody may still be referred to as a type of human antibody. Similarly, variants of a human antibody, for example those that have undergone affinity maturation, will also be understood to be "human antibodies" unless stated otherwise. In some embodiments, an antibody comprises, consists essentially of, or consists of a human, humanized, or chimeric monoclonal antibody.

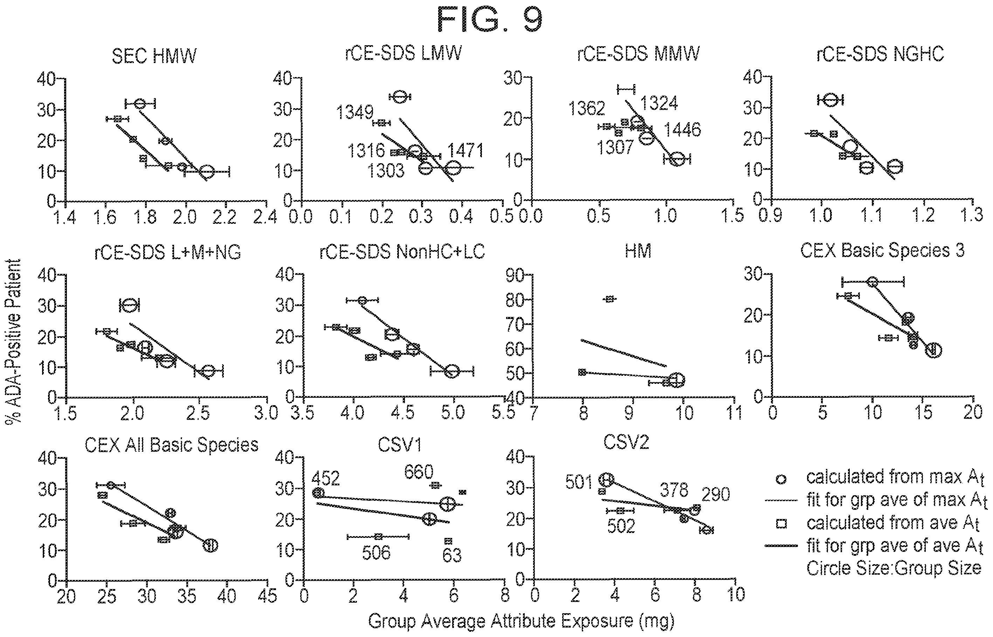

A "heavy chain" of an antigen binding protein (such as an antibody) includes a variable region ("VH"), and three constant regions: CH1, CH2, and CH3. Example heavy chain constant regions suitable for antigen binding proteins described herein, including example human IgG1, IgG2, IgG3, and IgG4 constant regions, are shown in FIGs. 9A-B. A "light chain" of an antigen binding protein (such as an antibody) includes a variable region ("VL"), and a constant region ("CL"). Human light chains include kappa chains and lambda chains. Example light chain constant regions suitable for antigen binding proteins described herein, including example human lambda and human kappa constant regions are shown in FIG. 9C.

In various aspects, the biological therapy is an antibody protein product. As used herein, the term "antibody protein product" refers to any one of several antibody alternatives which in various instances is based on the architecture of an antibody but is not found in nature. In some aspects, the antibody protein product has a molecular-weight within the range of at least about 12-150 kDa. In certain aspects, the antibody protein product has a valency (n) range from monomeric (n = 1), to dimeric (n = 2), to trimeric (n = 3), to tetrameric (n = 4), if not higher order valency. Antibody protein products in some aspects are those based on the full antibody structure and/or those that mimic antibody fragments which retain full antigen-binding capacity, e.g., scFvs, Fabs and VHH/VH (discussed below). The smallest antigen binding antibody fragment that retains its complete antigen binding site is the Fv fragment, which consists entirely of variable (V) regions. A soluble, flexible amino acid peptide linker is used to connect the V regions to a scFv (single chain fragment variable) fragment for stabilization of the molecule, or the constant (C) domains are added to the V regions to generate a Fab fragment [fragment, antigen-binding]. Both scFv and Fab fragments can be easily produced in host cells, e.g., prokaryotic host cells. Other antibody protein products include disulfide-bond stabilized scFv (ds-scFv), single chain Fab (scFab), as well as di- and multimeric antibody formats like dia-, tria-and tetra-bodies, or minibodies (miniAbs) that comprise different formats consisting of scFvs linked to oligomerization domains. The smallest fragments are VHH/VH of camelid heavy chain Abs as well as single domain Abs (sdAb). The building block that is most frequently used to create novel antibody formats is the single-chain variable (V)-domain antibody fragment (scFv), which comprises V domains from the heavy and light chain (VH and VL domain) linked by a peptide linker of ~15 amino acid residues. A peptibody or peptide-Fc fusion is yet another antibody protein product. The structure of a peptibody consists of a biologically active peptide grafted onto an Fc domain. Peptibodies are well-described in the art. See, e.g., Shimamoto et al.., mAbs 4(5): 586-591 (2012).

Biological therapies suitable for the methods described herein can include polypeptides, including those that bind to one or more of the following: These include CD proteins, including CD3, CD4, CD8, CD19, CD20, CD22, CD30, and CD34; including those that interfere with receptor binding. HER receptor family proteins, including HER2, HER3, HER4, and the EGF receptor. Cell adhesion molecules, for example, LFA-I, Mol, pl50, 95, VLA-4, ICAM-I, VCAM, and alpha v/beta 3 integrin. Growth factors, such as vascular endothelial growth factor ("VEGF"), growth hormone, thyroid stimulating hormone, follicle stimulating hormone, luteinizing hormone, growth hormone releasing factor, parathyroid hormone, Mullerian-inhibiting substance, human macrophage inflammatory protein (MIP-1alpha), erythropoietin (EPO), nerve growth factor, such as NGF-beta, platelet-derived growth factor (PDGF), fibroblast growth factors, including, for instance, aFGF and bFGF, epidermal growth factor (EGF), transforming growth factors (TGF), including, among others, TGF-$\alpha$ and TGF-$\beta$, including TGF-$\beta$I, TGF-$\beta$2, TGF-$\beta$3, TGF-$\beta$4, or TGF-$\beta$5, insulin-like growth factors-I and -II (IGF-I and IGF-II), des(I-3)-IGF-I (brain IGF-I), and osteoinductive factors. Insulins and insulin-related proteins, including insulin, insulin A-chain, insulin B-chain, proinsulin, and insulin-like growth factor binding proteins. Coagulation and coagulation-related proteins, such as, among others, factor VIII, tissue factor, von Willebrands factor, protein C, alpha-1-antitrypsin, plasminogen activators, such as urokinase and tissue plasminogen activator ("t-PA"), bombazine, thrombin, and thrombopoietin; (vii) other blood and serum proteins, including but not limited to albumin, IgE, and blood group antigens. Colony stimulating factors and receptors thereof, including the following, among others, M-CSF, GM-CSF, and G-CSF, and receptors thereof, such as CSF-1 receptor (c-fms). Receptors and receptor-associated proteins, including, for example, flk2/flt3 receptor, obesity (OB) receptor, LDL receptor, growth hormone receptors, thrombopoietin receptors ("TPO-R," "c-mpl"), glucagon receptors, interleukin receptors, interferon receptors, T-cell receptors, stem cell factor receptors, such as c-Kit, and other receptors. Receptor ligands, including, for example, OX40L, the ligand for the OX40 receptor. Neurotrophic factors, including bone-derived neurotrophic factor (BDNF) and neurotrophin-3, -4, -5, or -6 (NT-3, NT-4, NT-5, or NT-6). Relaxin A-chain, relaxin B-chain, and prorelaxin; interferons and interferon receptors, including for example, interferon-a, -β, and -y, and their receptors. Interleukins and interleukin receptors, including IL-I to IL-33 and IL-I to IL-33 receptors, such as the IL-8 receptor, among others. Viral antigens, including an AIDS envelope viral antigen. Lipoproteins, calcitonin, glucagon, atrial natriuretic factor, lung surfactant, tumor necrosis factor-alpha and -beta, enkephalinase, RANTES (regulated on activation normally T-cell expressed and secreted), mouse gonadotropin-associated peptide, DNAse, inhibin, and activin. Integrin, protein A or D, rheumatoid factors, immunotoxins, bone morphogenetic protein (BMP), superoxide dismutase, surface membrane proteins, decay accelerating factor (DAF), HIV envelope, transport proteins, homing receptors, addressins, regulatory proteins, immunoadhesins, antibodies. Myostatins, TALL proteins, including TALL-I, amyloid proteins, including but not limited to amyloid-beta proteins, thymic stromal lymphopoietins ("TSLP"), RANK ligand ("RANKL" or "OPGL"), c-kit, TNF receptors, including TNF Receptor Type 1, TRAIL-R2, angiopoietins, and biologically active fragments or analogs or variants of any of the foregoing.

Examples of biological therapies suitable for the methods described herein include antibodies such as infliximab, bevacizumab, cetuximab, ranibizumab, palivizumab, abagovomab, abciximab, actoxumab, adalimumab, afelimomab, afutuzumab, alacizumab, alacizumab pegol, ald518, alemtuzumab, alirocumab, altumomab, amatuximab, anatumomab mafenatox, anrukinzumab, apolizumab, arcitumomab, aselizumab, altinumab, atlizumab, atorolimiumab, tocilizumab, bapineuzumab, basiliximab, bavituximab, bectumomab, belimumab, bemarituzumab, benralizumab, bertilimumab, besilesomab, bevacizumab, bezlotoxumab, biciromab, bivatuzumab, bivatuzumab mertansine, blinatumomab, blosozumab, brentuximab vedotin, briakinumab, brodalumab, canakinumab, cantuzumab mertansine, cantuzumab mertansine, caplacizumab, capromab pendetide, carlumab, catumaxomab, cc49, cedelizumab, certolizumab pegol, cetuximab, citatuzumab bogatox, cixutumumab, clazakizumab, clenoliximab, clivatuzumab tetraxetan, conatumumab, crenezumab, cr6261, dacetuzumab, daclizumab, dalotuzumab, daratumumab, demcizumab, denosumab, detumomab, dorlimomab aritox, drozitumab, duligotumab, dupilumab, ecromeximab, eculizumab, edobacomab, edrecolomab, efalizumab, efungumab, elotuzumab, elsilimomab, enavatuzumab, enlimomab pegol, enokizumab, enoticumab, ensituximab, epitumomab cituxetan, epratuzumab, erenumab, erlizumab, ertumaxomab, etaracizumab, etrolizumab, evolocumab, exbivirumab, fanolesomab, faralimomab, farletuzumab, fasinumab, fbta05, felvizumab, fezakinumab, ficlatuzumab, figitumumab, flanvotumab, fontolizumab, foralumab, foravirumab, fresolimumab, fulranumab, futuximab, galiximab, ganitumab, gantenerumab, gavilimomab, gemtuzumab ozogamicin, gevokizumab, girentuximab, glembatumumab vedotin, golimumab, gomiliximab, gs6624, ibalizumab, ibritumomab tiuxetan, icrucumab, igovomab, imciromab, imgatuzumab, inclacumab, indatuximab ravtansine, infliximab, intetumumab, inolimomab, inotuzumab ozogamicin, ipilimumab, iratumumab, itolizumab, ixekizumab, keliximab, labetuzumab, lebrikizumab, lemalesomab, lerdelimumab, lexatumumab, libivirumab, ligelizumab, lintuzumab, lirilumab, lorvotuzumab mertansine, lucatumumab, lumiliximab, mapatumumab, maslimomab, mavrilimumab, matuzumab, mepolizumab, metelimumab, milatuzumab, minretumomab, mitumomab, mogamulizumab, morolimumab, motavizumab, moxetumomab pasudotox, muromonab-cd3, nacolomab tafenatox, namilumab, naptumomab estafenatox, narnatumab, natalizumab, nebacumab, necitumumab, nerelimomab, nesvacumab, nimotuzumab, nivolumab, nofetumomab merpentan, ocaratuzumab, ocrelizumab, odulimomab, ofatumumab, olaratumab, olokizumab, omalizumab, onartuzumab, oportuzumab monatox, oregovomab, orticumab, otelixizumab, oxelumab, ozanezumab, ozoralizumab, pagibaximab, palivizumab, panitumumab, panobacumab, parsatuzumab, pascolizumab, pateclizumab, patritumab, pemtumomab, perakizumab, pertuzumab, pexelizumab, pidilizumab, pintumomab, placulumab, ponezumab, priliximab, pritumumab, PRO 140, quilizumab, racotumomab, radretumab, rafivirumab, ramucirumab, ranibizumab, raxibacumab, regavirumab, reslizumab, rilotumumab, rituximab, robatumumab, roledumab, romosozumab, rontalizumab, rovelizumab, ruplizumab, samalizumab, sarilumab, satumomab pendetide, secukinumab, sevirumab, sibrotuzumab, sifalimumab, siltuximab, simtuzumab, siplizumab, sirukumab, solanezumab, solitomab, sonepcizumab, sontuzumab, stamulumab, sulesomab, suvizumab, tabalumab, tacatuzumab tetraxetan, tadocizumab, talizumab, tanezumab, taplitumomab paptox, tefibazumab, telimomab aritox, tenatumomab, tefibazumab, teneliximab, teplizumab, teprotumumab, tezepelumab, TGN1412, tremelimumab, ticilimumab, tildrakizumab, tigatuzumab, TNX-650, tocilizumab, toralizumab, tositumomab, tralokinumab, trastuzumab, TRBS07, tregalizumab, tucotuzumab celmoleukin, tuvirumab, ublituximab, urelumab, urtoxazumab, ustekinumab, vapaliximab, vatelizumab, vedolizumab, veltuzumab, vepalimomab, vesencumab, visilizumab, volociximab, vorsetuzumab mafodotin, votumumab, zalutumumab, zanolimumab, zatuximab, ziralimumab, or zolimomab aritox.

In some embodiments, the biological therapy is a BiTE® molecule. BiTE® molecules are engineered bispecific antigen binding constructs which direct the cytotoxic activity of T cells against cancer cells. They are the fusion of two single-chain variable fragments (scFvs) of different antibodies, or amino acid sequences from four different genes, on a single peptide chain of about 55 kilodaltons. One of the scFvs binds to T cells via the CD3 receptor, and the other to a tumor cell via a tumor specific molecule. Blinatumomab (BLINCYTO®) is an example of a BiTE® molecule, specific for CD19.

BiTE® molecules that are modified, such as those modified to extend their half-lives, can also be used in the disclosed methods. In various aspects, the polypeptide is an antigen binding protein, e.g., a BiTE® molecule. In some embodiments, an antibody protein product comprises a BiTE® molecule.

In some embodiments, the biological therapy is in a formulation. The formulation may be a pharmaceutically acceptable formulation. The formulation may comprise the biological therapy together with a pharmaceutically acceptable diluent, carrier, solubilizer, emulsifier, preservative, and/or adjuvant.

Acceptable formulation materials for biological therapies as described herein preferably are nontoxic to recipients at the dosages and concentrations employed. In certain embodiments, the pharmaceutical composition may contain formulation materials for modifying, maintaining or preserving, for example, the pH, osmolality, viscosity, clarity, color, isotonicity, odor, sterility, stability, rate of dissolution or release, adsorption or penetration of the composition. In such embodiments, suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates or other organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides; and other carbohydrates (such as glucose, sucrose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. See, e.g., REMINGTON'S PHARMACEUTICAL SCIENCES, 18" Edition, (A. R. Genrmo, ed.), 1990, Mack Publishing Company.

A suitable vehicle or carrier for the formulation may be water for injection, physiological saline solution or artificial cerebrospinal fluid, possibly supplemented with other materials common in compositions for parenteral administration. Neutral buffered saline or saline mixed with serum albumin are further exemplary vehicles. In specific embodiments, pharmaceutical compositions comprise Tris buffer of about pH 7.0-8.5, or acetate buffer of about pH 4.0-5.5, and may further include sorbitol or a suitable substitute therefor.

The formulation components are present preferably in concentrations that are acceptable to the site of administration. In certain embodiments, buffers are used to maintain the composition at physiological pH or at a slightly lower pH, typically within a pH range of from about 5 to about 8. Including about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, and about 8.0.

It is noted that some biological therapies may be self-administered by subjects, either directly, or via an automated injector, while some may be administered to the subject by another individual such as a health care provider. As such, a subject "receiving and administration" or "being administered" a biological therapy to a subject, and variations of these root terms, as used herein, may refer to the biological therapy being self-administered by the subjects to themselves (either directly, or via an apparatus such as an automated injector), and/or by other individuals, such as health care providers, to the subject.

## Methods of manufacturing a biological therapy

It is contemplated that methods described herein may be used to assess whether molecular attributes impact efficacy of a biological therapy, and/or impact a safety profile of a biological therapy. The methods can comprise determining an estimated actual level of molecular attribute exposure at the time that a subject receives an administration of a biological therapy. Such methods can identify suitable molecular attribute levels or ranges for manufacturing, lot release, and/or time of administration.

In some embodiments, a method of manufacturing a biological therapy is described. The method can comprise detecting a level of a molecular attribute of the biological therapy in a formulation at one or more timepoints under storage conditions (optionally, at two or more timepoints under storage conditions). Optionally, for example instances in which there is no change in molecular attributes upon storage (for example for some drug products that are stored frozen), it may be sufficient to detect the level of molecular attribute at one timepoint. It is further contemplated, that for levels of molecular attributes that may change during storage, detecting the level of the molecular attribute at two more timepoints under storage conditions can be used to calculate a rate of change of the molecular attribute as described herein. By way of example, the timepoints may comprise the time of manufacture. By way of example, the timepoints may comprise the time of manufacture and at least one other timepoint. The method can comprise determining a rate of change of the molecular attribute under the storage conditions (it is contemplated that for some molecular attribute of some biological therapies under some storage conditions, for example some biological therapies that are stored frozen, the rate of change may be calculated as zero). The method can comprise obtaining data on *in vivo* safety and/or efficacy of the biological therapy for subjects that have received an administration of the biological therapy. The method can comprise estimating a level of molecular attribute exposure received by the subjects at the time of said administration, based on (i) the rate of change of the molecular attribute during storage and (ii) a duration that the biological therapy in the formulation was under the storage conditions prior to said administration. The estimating may also take into account (iii) the level of the molecular attribute at the time of manufacture and/or at lot release, and/or (iv) the dose of the biological product administered to the subject. By way of example, the estimating may use equation 1 or equation 2 or equation 3. The method can comprise determining a presence or absence of a correlation between the estimated level of molecular attribute exposure received by the subjects and the safety and/or efficacy data for the biological therapy. If the correlation is absent, the method can comprise manufacturing production lots of the biological therapy comprising the molecular attribute at or below a specified permissible level of the molecular attribute based on the estimated level of molecule attribute exposure. For example, the specified permissible level may be a level of the molecular attribute that is calculated to yield a level of attribute exposure at end-of-shelf that is less than or equal to the highest estimated level of molecular attribute exposure in subjects. For example, the specified permissible level may be a level of the molecular attribute that is calculated to produce a level of attribute exposure at end-of-shelf that is less than or equal to 90 - 100% of the highest estimated level of molecular attribute exposure in subjects. Accordingly, the permissible level is expected to yield levels of attribute exposure that are shown to be safe and effective in subjects throughout the shelf life of the biological therapy. Production lots with levels of the molecular attribute that exceed the specified permissible level may be rejected.

If the correlation is present, the method can further comprise setting a specification for levels of the molecular attribute at the time of manufacture to not exceed a maximum permissible level of the molecular attribute of the biological therapy. The maximum permissible level of the molecular attribute can be based on the highest estimated levels of molecular attribute exposure in subjects that are not associated with adverse events and/or inhibition of efficacy of the biological therapy. The method of manufacturing can further comprise rejecting production lots of the biological therapy comprising levels of the molecular attribute exceeding the maximum permissible level (and thus outside of the specification). Production lots with levels of the molecular attribute that do not exceed the specified maximum permissible level may be accepted. For example, the specified maximum permissible level may be a level of the molecular attribute that is calculated to produce a level of attribute exposure at end-of-shelf that is less than or equal to the highest estimated level of molecular attribute exposure that is not associated with adverse events and/or an inhibition of efficacy in subjects. The calculation may use equation 1 or equation 2 or equation 3, and may take into account the rate of change of the molecular attribute in formulation during storage, and the dose of molecular attribute to be administered to a subject. For example, the specified maximum permissible level may be a level of the molecular attribute that is calculated to produce a level of attribute exposure at end-of-shelf that is less than or equal to 90 - 100% of the highest estimated level of molecular attribute exposure that is not associated with adverse events and/or an inhibition of efficacy in subjects.

As used herein a "permissible level" of molecular attribute refers to a level of molecular attribute of a biological therapy in formulation that is within specification at the time of manufacture. As described herein, the permissible level may be calculated to yield a specified level of molecular attribute exposure (to a subject who would be administered the biological therapy in formulation) at end-of-shelf or expiration that is less than or equal to an estimated level of molecular attribute exposure that was not associated with adverse events and/or loss of efficacy (this may be referred to as a permissible level "based on" the estimated level of attribute exposure). Thus, permissible levels of molecular attributes can provide confidence that the level of molecular attribute exposure from the biological therapy in formulation is safe and effective when administered to a subject, even if it is administered very close to its end-of-shelf or expiration date. A "maximum permissible level" refers to a scenario when there is a correlation between the estimated level of molecular attribute exposure and safety and/or efficacy data. The "maximum permissible level" refers to the highest level of molecular attribute in formulation that yields a level of molecular attribute exposure (to a subject who would be administered the biological therapy in formulation) at end-of-shelf or expiration that is less than or equal to the highest level of molecular attribute exposure *not* associated with adverse events. The permissible level or maximum permissible level may be calculated using equation 1 or equation 2 or equation 3 based on the estimated level of attribute exposure not associated with adverse events, the duration until end-of-shelf or expiration, and rate of change of the level of molecular attribute, and the dose of the biological therapy. That is, using the level of molecular attribute exposure that was not associated with adverse events and/or inhibition of efficacy, the rate of change in the molecular attribute in formulation, and this duration, the permissible level (if applicable, the maximum permissible level) may be determined. Thus, if a biological therapy is manufactured with a molecular attribute at or below a permissible level (if applicable a maximum permissible level), it can be expected that at the time of administration to a subject, the estimated level of molecular attribute exposure to the subject will be at or below a level that is not associated with adverse events and/or loss of efficacy. As used herein, a permissible level or maximum permissible level "based on" an estimated level of a molecular attribute exposure refers to the permissible level (or maximum permissible level) calculated to yield no more than the estimated level of molecular attribute at end-of-shelf or expiration. If more than one dose is suitable for a biological therapy in formulation, the highest suitable dose may be used to calculate the permissible level or maximum permissible level "based on" the estimated level of a molecular attribute (as lower doses will have even lower levels of the molecular attribute exposure). By way of example, the estimated level of attribute exposure may be selected to fall within a confidence interval for a distribution or a spread of estimated molecular attribute exposure levels that were determined for a group of subjects. It is contemplated that an estimated level of attribute exposure received by subjects does not necessarily imply that every subject received the same numerical level of attribute exposure. Rather, the estimated level of attribute exposure received by those subjects may comprise a distribution of estimated attribute levels received by individual subjects. Thus, the maximum permissible level may be selected using a confidence interval so that there is at least an 85%, 90%, 95%, 97%, or 99% probability that the level of attribute exposure at end of shelf is less than or equal to the highest level of attribute exposure that was not associated with a loss of safety or efficacy. It is further contemplated that the actual value of the permissible or maximum permissible level "based on" the level of molecular attribute exposure may undergo rounding. The rounding may provide administrative or mathematical convenience, such as rounding to one, two, or three significant figures in a suitable unit for measuring the molecular attribute. For added caution, the rounding may be a rounding down. For example, the permissible level or maximum permissible level of attribute "based on" the estimated level of attribute exposure may be calculated to yield a level of attribute exposure at end-of-shelf that is no more than 99%, 97%, 95%, 90%, 85%, or 80% of the reference level of attribute exposure (that was not associated with a loss of safety and/or efficacy) calculated using Equation 1 or 2 as described herein. This rounding down can provide additional assurance that the level of attribute exposure from the end-of-shelf biological therapy will still be safe and/or effective.

In some embodiments, production lots are manufactured with levels of more than one molecular attribute, each at or below its permissible (or maximum permissible) level. For example, the production lots may be manufactured with at least one, two, three, four, five, six, seven, eight, nine, or ten molecular attributes, including ranges between any two of the listed values, for example one - ten, one - five, two - ten, two - five, three - ten, three - five, or five - ten molecular attributes, each of which is at or below a specified permissible (or maximum permissible) level.

*Manufacturing techniques*

Methods for manufacturing biological therapies as described herein (e.g., therapeutic proteins) may utilize recombinant DNA technology. Recombinant DNA methods for producing therapeutic proteins such as antibodies or antibody protein products are well-known. DNA may encode the therapeutic protein. For example, DNA may encode the antibodies, for example, DNA encoding a VH domain, a VL domain, a single chain variable fragment (scFv), or fragments and combinations thereof (target polynucleotides), can be inserted into a suitable expression vector, which can then be transfected into a suitable host cell, such as Escherichia coli cells, COS cells, Chinese Hamster Ovary (CHO) cells, or myeloma cells that do not otherwise produce an antibody, to obtain the desired antibodies.

Suitable expression vectors are known in the art, containing, for example a polynucleotide that encodes the target polypeptide linked to a promoter. Such vectors can include the nucleotide sequence encoding the constant region of the antibody molecule, and the variable domain of the antibody can be cloned into such a vector for expression of the heavy chain, the entire light chain, or both the entire heavy and light chains (or fragments thereof). The expression vector can be transferred to a host cell by conventional techniques, and the transfected cells can be cultured to produce the antibodies.

Any cell line that can express, or is engineered to express, proteins such as functional antibody or antibody fragments, can be used. For example, suitable mammalian cell lines include immortalized cell lines available from the American Type Culture Collection (Manassas, VA), including Chine Hamster Ovary (CH)) cells, HeLa cells, baby hamster kidney (BHK) cells, monkey kidney cells (COS), human hepatocellular carcinoma cells (e.g., Hep G2), and human epithelial kidney 293 cells. Furthermore, cell lines or host systems can be chosen to ensure correct modification and processing of antibodies. Eukaryotic host cells that possess the cellular machinery for proper processing of the primary transcript, glycosylation, and phosphorylation of the gene product can be used. These include CHO, VERY, BHK, Hela, COS, MDCK, 293, 3T3, W138, BT483, Hs578T, HTB2, BT20 and T47D, NS0 (a murine myeloma cell line that does not endogenously produce any functional immunoglobulin chains), SP20, CRL7030 and HsS78Bst cells. Human cell lines developed by immortalizing human lymphocytes can also be used. The human cell line PER.C6® (Janssen; Titusville, NJ) can be used to recombinantly produce monoclonal antibodies. Examples of non-mammalian cells that can also be used include insect cells (e.g., Sf21/Sf9, Trichoplusia ni Bti-Tn5bl-4), or yeast cells (e.g., *Saccharomyces* (such as S. *cerevisiae, Pichia,* etc.), plant cells, or chicken cells.

Proteins such as antibodies can be stably expressed in a cell line using conventional methods. Stable expression can be used for long-term, high-yield production of recombinant proteins. For stable expression, host cells can be transformed with an appropriately engineered vector that includes expression control elements (e.g., promoter, enhancer, transcription terminators, polyadenylation sites, etc.), and a selectable marker gene. Methods for producing stable cell lines with a high yield are known in the art and reagents are available commercially. Transient expression can also be accomplished using conventional methods.

A cell line expressing a protein such as an antibody can be maintained in cell culture medium and under culture conditions that result in the expression and production of the antibodies. Cell culture media can be based on commercially available media formulations, including, for example, DMEM or Ham's F12. In addition, the cell culture media can be modified to support increases in both cell growth and biologic protein expression. Of course, cell culture medium can be optimized for a specific cell culture, including cell culture growth medium which is formulated to promote cellular growth or cell culture production medium which is formulated to promote recombinant protein production.

Many cell culture media and cell culture nutrients and supplements are known. For example, suitable basal media include Dulbecco's Modified Eagle's Medium (DMEM), DME/F12, Minimal Essential Medium (MEM), Basal Medium Eagle (BME), RPMI 1640, F-10, F-12, a-Minimal Essential Medium (a-MEM), Glasgow's Minimal Essential Medium (G-MEM), PF CHO, and Iscove's Modified Dulbecco's Medium. Other examples of basal media that can be used include BME Basal Medium, Dulbecco's Modified Eagle Medium.

The basal medium may be serum-free, meaning that the medium contains no serum (e.g., fetal bovine serum (FBS)) or animal protein-free media or chemically-defined media. The basal medium can be modified in order to remove certain non-nutritional components found in basal media, such as various inorganic and organic buffers, surfactant(s), and sodium chloride. The cell culture medium can contain a basal cell medium (modified or not), and at least one of the following: iron source, recombinant growth factor; buffer; surfactant; osmolarity regulator; energy source; and non-animal hydrolysates. In addition, the modified basal cell medium can optionally contain amino acids, vitamins, or a combination of both amino acids and vitamins. A modified basal medium can further contain glutamine, e.g., L-glutamine, and/or methotrexate.

Once an therapeutic protein (e.g., an antibody or antibody protein product) has been produced, it can be purified by conventional methods, for example, by chromatography (e.g., ion exchange, affinity, particularly by affinity for the specific antigens, Protein A, Protein G, or sizing column chromatography), centrifugation, differential solubility, or by any other standard technique for the purification of proteins. Further, the protein can be fused to heterologous polypeptide sequences ("tags") to facilitate purification.

The purified protein is typically formulated with an excipient to produce a sterile solution that can be injected or infused. For example, the purified protein may be formulated in a formulation as described herein. Formulation may be followed by filling, packaging, storage, delivery, and final preparation immediately prior to administration to the subject.

## Methods of developing a manufacturing process for biological therapy

It is contemplated that methods described herein may also be used to develop methods for manufacturing a biological therapy. In some embodiments, a method of developing a manufacturing process for biological therapy is described. The method can comprise detecting a level of a molecular attribute of the biological therapy in a formulation at one or more timepoints under storage conditions (optionally at two or more timepoints under storage conditions). For example, the timepoints may include the time of manufacture. For example, the timepoints may include the time of manufacture, and at least one other timepoint. The method can comprise determining a rate of change of the molecular attribute under the storage conditions. The method can comprise obtaining data on safety and/or efficacy of the biological therapy in subjects that have received an administration of the biological therapy. The method can comprise estimating a level of molecular attribute exposure received by the subjects at the time of administration, based on (i) the rate of change of the molecular attribute during storage of the biological therapy in the formulation and (ii) a duration that the biological therapy in the formulation was under the storage conditions prior to said administration. The estimating may also take into account (iii) the level of the molecular attribute at the time of manufacture and/or at lot release, and/or (iv) the dose of the biological product administered to the subject. By way of example, the estimating may use equation 1 or equation 2 or equation 3. The method can comprise determining a presence or absence of a correlation between the estimated level of molecular attribute exposure and the safety and/or efficacy data of the biological therapy.

If (a), the correlation is absent, the method can comprise establishing the manufacturing process to produce levels of the molecular attribute at or below a specified permissible level based on the estimated level of molecular attribute exposure. For example, the specified permissible levels may be a level of the molecular attribute that is calculated to yield a level of attribute exposure at end-of-shelf that is less than or equal to the highest estimated level of molecular attribute exposure in subjects. The calculation may use equation 1 or equation 2 or equation 3. For example, the specified permissible level may be a level of the molecular attribute that is calculated to produce a level of attribute exposure at end-of-shelf that is less than or equal to 90-100% of the highest estimated level of molecular attribute exposure in subjects. Production lots with levels of the molecular attribute that exceed the specified permissible level may be rejected.

If (b), the correlation is present, the method can comprise establishing the manufacturing process to produce levels of the molecular attribute at or below a specified a maximum permissible level of the molecular attribute based on the highest level of the molecular attribute that is not associated with adverse events and/or inhibition of efficacy of the biological therapy. For example, the specified maximum permissible level may be a level of the molecular attribute that is calculated to produce a level of attribute exposure at end-of-shelf that is less than or equal to the highest estimated level of molecular attribute exposure that is not associated with adverse events and/or an inhibition of efficacy in subjects. The calculation may use equation 1 or equation 2 or equation 3. For example, the specified maximum permissible level may be a level of the molecular attribute that is calculated to produce a level of attribute exposure at end-of-shelf that is less than or equal to 90 - 100% of the highest estimated level of molecular attribute exposure that is not associated with adverse events and/or an inhibition of efficacy in subjects. The permissible level or maximum permissible level may be part of a specification at time of manufacture or lot release.

## Methods of assessing clinical impact of a molecular attribute of a biological therapy

In some embodiments, a method of assessing a clinical impact of molecular attributes are described. Such method may further be used in a method of manufacturing a biological therapy, and in a method of developing a manufacturing process for a biological therapy as described herein. The method can comprise detecting a level of a molecular attribute of the biological therapy in a formulation at one or more timepoints under storage conditions. The level of attribute exposure can be detected at two or more timepoints under the storage conditions. By way of example, the timepoints for detecting the level of molecular attribute may include the time of manufacture or at least one other timepoint. By way of example, the two or more timepoints for detecting the level of molecular attribute may include the time of manufacture and at least one other timepoint. The method can comprise determining a rate of change of the molecular attribute under the storage conditions. The method can comprise obtaining data on safety and/or efficacy of the biological therapy in subjects that have received an administration of the biological therapy. The method can comprise estimating a level of molecular attribute exposure received by the subjects at the time of said administration, based on (i) the rate of change of the molecular attribute during storage of the biological therapy in the formulation and (ii) a duration that the biological therapy in the formulation was under the storage conditions prior to said administration. The estimating may also take into account (iii) the level of the molecular attribute at the time of manufacture and/or at lot release, and/or (iv) the dose of the biological product administered to the subject. By way of example, the estimating may use equation 1 or equation 2 or equation 3 as described herein. The method can comprise determining a presence or absence of a correlation between the estimated molecular attribute exposure and the safety and/or efficacy of the biological therapy. If (a) the correlation is absent, it can be determined that the molecular attribute impacts neither clinical safety nor efficacy of the biological therapy. If (b) the correlation is present, it can be determined that the molecular attribute impacts clinical safety and/or efficacy of the biological therapy.

If (a), the correlation is absent, the method may further comprise setting a specification for permissible levels of the molecular attribute of the biological therapy, in which the permissible levels of the molecular attribute are based on the highest estimated levels of the molecular attribute received by the subjects. For example, the specification for permissible levels may be a level of the molecular attribute at the time of manufacture that is calculated to yield a level of attribute exposure at end-of-shelf that is less than or equal to the highest estimated level of molecular attribute exposure in subjects. For example, the specification of permissible levels may be a level of the molecular attribute that is calculated to produce a level of attribute exposure at end-of-shelf that is less than or equal to 90 - 100% of the highest estimated level of molecular attribute exposure in subjects. The specification may be used for manufacturing and/or release testing of the biological therapy. If a lot or product of the biological therapy comprises the molecular attribute at a level that exceeds the permissible level, the lot or product may be considered out of specification and rejected. The biological therapy may be manufactured according to the specification.

If (b) the correlation is present, the method may further comprise setting a specification for maximum permissible levels of the molecular attribute of the biological therapy, in which the maximum permissible levels of the molecular attribute are based on levels of the highest estimated molecular attribute exposure that were not associated with adverse events and/or inhibition of efficacy of the biological therapy. The specification may be used for manufacturing and/or release testing of the biological therapy. For example, the specification for the maximum permissible level may be a level of the molecular attribute that is calculated to produce a level of attribute exposure at end-of-shelf that is less than or equal to the highest estimated level of molecular attribute exposure that is not associated with adverse events and/or an inhibition of efficacy in subjects. The calculation may use equation 1 or equation 2 or equation 3. For example, the specified maximum permissible level may be a level of the molecular attribute that is calculated to produce a level of attribute exposure at end-of-shelf that is less than or equal to 90 - 100% of the highest estimated level of molecular attribute exposure that is not associated with adverse events and/or an inhibition of efficacy in subjects. If a production lot or product of the biological therapy comprises the molecular attribute at a level that exceeds the maximum permissible level, the lot or product may be considered out of specification and rejected. The biological therapy may be manufactured according to the specification.

## Further Aspects of Methods

Any of the methods described herein may comprise one or more further aspects.

In some embodiments, for any method described herein, the estimating is further based on (iii) a dose of the biological therapy in said administration, and (iv) an amount of the molecular attribute measured at time of manufacture and/or lot release. Regarding (iii), it is noted that a greater dose of biological product will administer a larger quantity of a molecular attribute than a smaller dose having the same percent content of the molecular attribute. It is also contemplated that in some instances, the level of the molecular attribute at time of manufacture and/or the dose may not vary appreciably, and thus the estimate can be performed from (i) and (ii) alone. For example, the manufacturing process may be well-established and tightly controlled and shown to yield consistent levels of a molecular attribute at the time of manufacture. For example, the biological therapy may only be administered at a single dose, so that there is no need to weigh the calculation by dose.

In some embodiments, for any method described herein, the level of molecular attribute of the biological therapy in the formulation is measured at one or more timepoints at or after time of manufacture, for example, at least one, two, three, four, five, six, seven, eight, nine, or ten timepoints, including ranges between any two of the listed values, for example one-two timepoints, one-five timepoints, or one-ten timepoints. Without being limited by theory, it is contemplated that for some biological therapies in formulation, for which the levels of molecular attribute do not change during storage, as may be the case for some biological therapies in formulation that are stored frozen, it may be sufficient to measure the level of molecular attribute at a single timepoint. In some embodiments, for any method described herein, the level of molecular attribute of the biological therapy in the formulation is measured at two or more timepoints, for example at least two, three, four, five, six, seven, eight, nine, or ten timepoints, including ranges between any two of the listed values, for example, two-five timepoints, two-ten timepoints, three-five timepoints, three-ten timepoints, or five-ten timepoints. The levels of the molecular attribute at the two or more timepoints can be used to calculate a rate of change in the levels of the molecular attribute under storage conditions. The timepoints may be at or after the time of manufacture. In some embodiments, for any method described herein, the two or more timepoints comprise a time of manufacture. In some embodiments, for any method described herein, the two or more timepoints comprise a time of manufacture, and at least one subsequent timepoint. In some embodiments, for any method described herein, the two or more timepoints comprise a time of manufacture, and at least two subsequent timepoints.

In some embodiments, for any method described herein, estimating the level of molecular attribute exposure comprises using an equation as described herein. In some embodiments, estimating the level of the molecular attribute exposure comprises using equation 1:

$$A_t = (\%A_0 + \%A_\Delta \times t)D \qquad\qquad (1)$$

in which, $A_t$ is the estimated level of molecular attribute exposure, $\%A_0$ is the percentage of attribute at the time of lot release, $\%A_\Delta$ is rate of change in the percentage of attribute level, t is the storage time between lot release and treatment administration, and D is the dose strength in the dimension of weight of active pharmaceutical ingredient associated with each treatment.

In some embodiments, estimating the level of the molecular attribute exposure comprises using equation 2:

$$A_t = \left\{ \%A_0 + \sum_{i=1}^{n} \left( \%A_{\Delta_i} \times t_i \right) \right\} \times D$$

$$(2)$$

in which $A_t$ is the estimated level of molecular attribute exposure, $\%A_0$ is the percentage of the molecular attribute at time of lot release, $\%A_{\Delta i}$ is the rate of change of the percentage of the molecular attribute level over time at a given storage condition, $t_i$ is the time of storage at the given condition, and D is a dose strength of the administration.

In some embodiments, estimating the level of molecular attribute exposure comprises using equation 3:

$$\%A_{\text{rel.}} = \frac{A_t}{D} \times 100\%$$

$$(3)$$

in which $\%A_{\text{rel.}}$ is the percentage of the relative level of the molecular attribute exposure with respect to the dosage, where $A_t$ is the level of the molecular attribute exposure computed using the equation 1 or 2, and $D$ is the dose strength in the dimension of weight of active pharmaceutical ingredient associated with each treatment. It is noted that equation 3 can account for the relative level of molecular attribute exposure relative to dose strength. Without being limited by theory, if a molecular attribute impacts the efficacy, it can be inferred that non-attributes may also be impacting efficacy.

For example, the relative concentration of active pharmaceutical ingredient (API) with respect to dosage strength may impact efficacy. Accordingly, the % molecular attribute relative to the dosage strength can be useful for measuring the effect of a molecular attribute on efficacy. As such, in methods of some embodiments, equation 3 can be used when a presence or absence of a correlation between the estimated level of molecular attribute exposure and efficacy data is determined.

In some embodiments, for methods described herein, estimating the level of molecular attribute exposure received by the subject comprises assigning an estimated attribute exposure level as follows:

(A) $A_t$ is first determined using Equation 1 or Equation 2 or Equation 3 for all relevant treatments as described below.

(1) For patients who do not show the clinical outcome of interest throughout the course of the clinical studies, the relevant treatments are all treatments administered to the patients throughout the study.
(2) For patients showing the clinical outcome, the relevant treatments are all treatments administered to the patients prior to the occurrence of the clinical outcome.
(3) For patients who develop the clinical outcome prior to receiving any treatment, no relevant treatment exists. $A_t$ is 0 for these patients.
(4) For biological therapies administered via continuous infusion, $A_t$ that occurs during the relevant portion of the infusion may be determined as follows (for biological therapies not administered via continuous fusion, item (4) may be omitted):

a. For infusion continuously administered for longer than 12 or 24 hours, $A_t$ per each 12 or 24-hour-long segment of the relevant infusion prior to occurrence of the clinical outcome is determined.
b. For infusions that are less than 12 hours long (or 24 hours long, as applicable), $A_t$ associated with each of the relevant infusion prior to the occurrence of the clinical outcome is determined.
c. For either of the above 2 cases (a and b), if the clinical outcome occurs during an infusion, $A_t$ associated with the portion of the infusion prior to the occurrence of the clinical outcome is determined.

(B) Once $A_t$ for all relevant treatments are determined for each patient with or without the clinical outcome, the average, or maximum, of all $A_t$ associated with the relevant treatments or relevant portion of infusions is calculated as defined above.

It is noted that additional mathematical operations may be applied to further refine the determination of a correlation between the level of molecular attribute exposure, and the data on safety and efficacy. In some embodiments, for any method described herein, the correlation comprises a weighted correlation between attribute exposure and adverse event occurrences. The weighting may use the size of each groups of subjects. Without being limited by theory, it is contemplated that if subjects experience adverse events in a multi-dose regimen of a biological therapy, those subjects may stop using the biological therapy. In the aggregate, there may be fewer data for subjects experiencing adverse events than for those subjects who did not experience adverse events, which may impact the determination of a correlation (or lack thereof). Accordingly, data may optionally be weighted to account for subjects who discontinued a multi-dose regimen of a biological therapy before completing the regiment, for example subjects who experienced adverse events.

It is further contemplated that for different subjects, the biological therapy in the formulation may be under storage conditions for different durations for different subjects. As such, there may not be a single duration for which the biological therapy in the formulation was under storage conditions. Rather, different durations may be applied to different administrations of the biological therapy, as appropriate. Estimating the level of the molecular attribute exposure may account for these different durations by selecting an appropriate time of storage ($t_i$) in each instance. Thus, in the method of some embodiments, the biological therapy was under the storage conditions for different durations in the administrations to different subjects.

It is further contemplated that for methods as described herein, the administration of the biological therapy may comprise two or more administration events, for example at least two, three, four, five, or ten administration events, including ranges between any two of the listed values, such as two-three, two-five, two-ten, three-five, three-ten, or five-ten administration events. According, in some embodiments, the estimated level of attribute exposure received by the subjects at the time of administration is a maximum or an average of the two or more administration events.

It is further contemplated that for methods as described herein, the administration of the biological therapy may comprise a continuous infusion. The continuous infusion may be, for example, over a period of hours or days. In some embodiments, the administration comprises a continuous infusion, and estimating the level of attribute exposure comprises calculating an estimated level of molecular attribute exposure during two or more intervals of the continuous infusion, such 12-hour or 24-hour intervals.

Methods described herein may utilize clinical study data. These include, but are not limited to, the subject number, clinical outcome information (date, time and extent (severity or grade)), treatment lot number, treatment (date, time and duration). Data may be grouped according to clinical outcome (e.g., clinical endpoints and/or adverse events). Adverse event may be indicative of safety data, and clinical endpoints may be indicative of efficacy data. In clinical outcome-based grouping, subjects are grouped according to the occurrence of the clinical outcome with the severity or extent of interest. By way of example, to analyze the impact of a molecular attribute on the severity of a safety adverse event, the subjects are grouped according to the occurrence of the AE with the severity grade of interest (e.g. The severe fever-positive group would include the patients who had a fever with grade 3 or higher, whereas the severe fever-negative group would include the subjects who did not have a fever with grade 3 or higher). Then, the estimated level of attribute exposure for each subject is determined as described.

By way of example, safety data for the methods described herein may comprise adverse event data. The adverse events may comprise treatment-related adverse events. For example, adverse events related to treatment with a biological therapy may comprise immune adverse events, such as an immune response against the biological therapy (e.g., anti-drug antibody (ADA)). Additional examples of adverse events include anemia, cytokine release syndrome (CRS), fever, infusion related reaction (IRR), lymphocytopenia, or neurological events. In some embodiments, for any method described herein, the safety data comprise adverse event data. By way of example, the safety data may comprise an adverse event time course.

By way of example, efficacy data for the methods described herein may comprise clinical evidence of efficacy, for example, treatment, prevention, amelioration, or delayed onset of a disease or disorder. Clinical endpoint data may be indicative of efficacy. Accordingly, in some embodiments, efficacy data comprise clinical endpoint data.

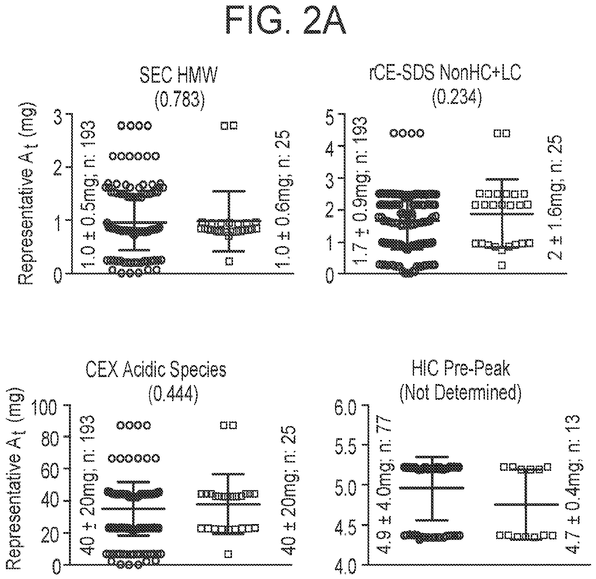

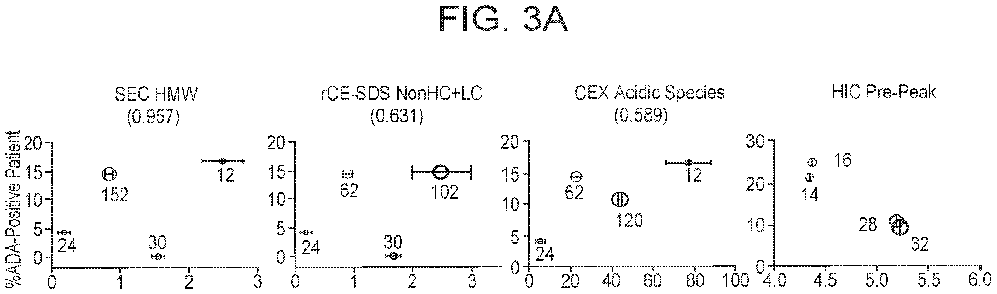

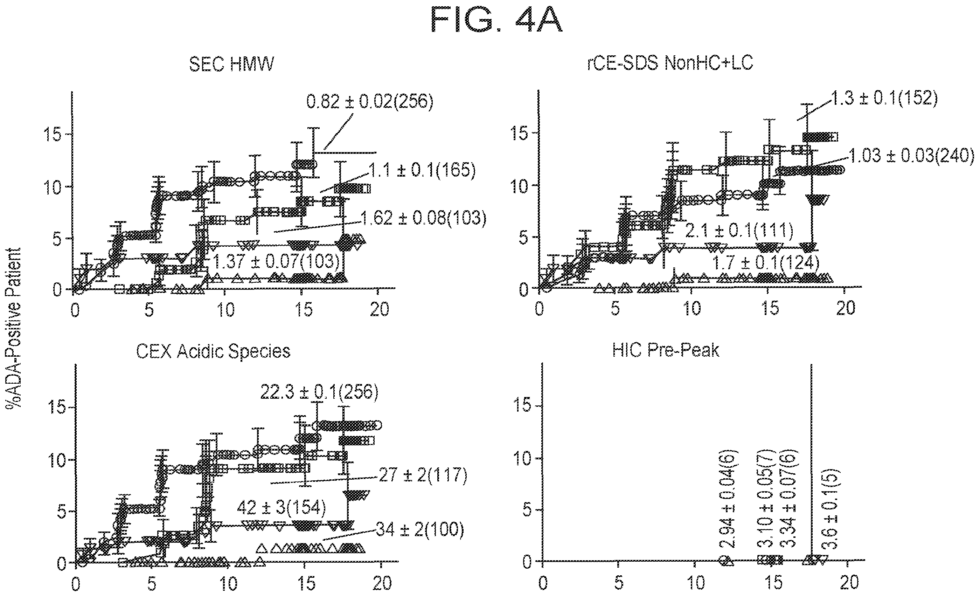

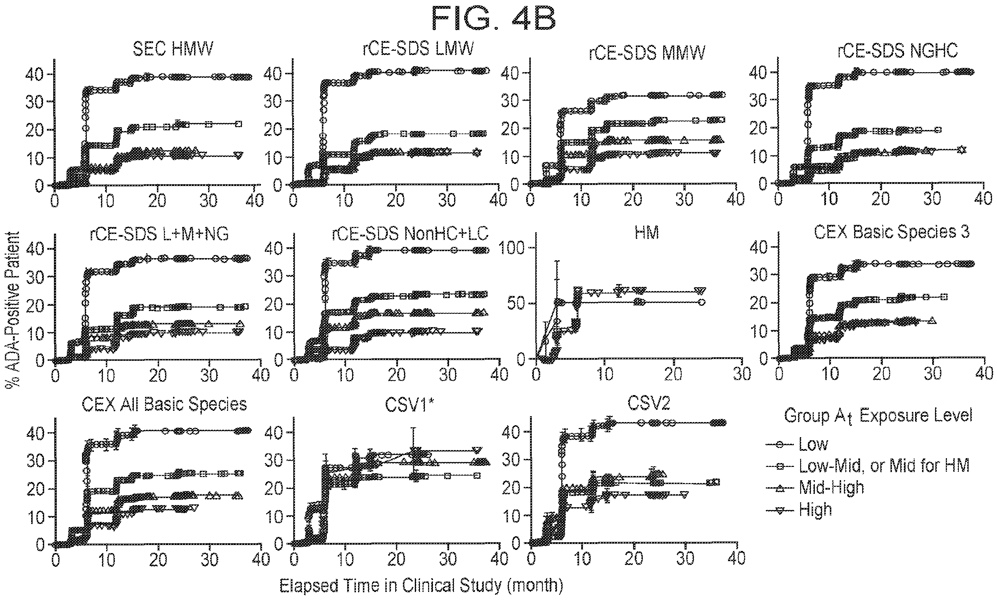

It is contemplated that for any of the methods herein, presence or absence of a correlation between the estimated level of molecular attribute exposure and the safety and/or efficacy data for the biological therapy may be calculated using statistical methods. For example, a linear regression correlation between the rate of clinical event from safety and/or efficacy data and attribute exposure level may be calculated, and p-values may be determined (Figs. 3A, 3B, 7B and 9). For example, the p values may be calculated from t-tests or F-tests (Figs. 2A, 2B, 5A, 5B, 6, 7A, 8, 10A and 10B). For example, Log-rank or Mantel-Cox test could be performed to calculate p-values for testing the earlier clinical safety event onsets for clinical subject groups with higher levels of the attribute exposures (Figs. 4A and 4B).

In some implementations of any of the methods described herein, a Bayesian estimation approach is used. The Bayesian estimation approach may be used for determining a presence or absence of a correlation between the estimated level of molecular attribute exposure and the safety and/or efficacy data for the biological therapy. Due to the stepwise learning nature of a Bayesian approach, all evidences may be fully used *a priori* in an unbiased way. In particular, when applying a Bayesian estimation approach for determining a presence or absence of a correlation between the estimated level of molecular attribute exposure and the safety and/or efficacy data for the biological therapy, the method may modify the probability density function of the clinical impact of each attribute to take a certain value whenever a new piece of evidence comes in, until all evidences are used. The probability of each attribute to be associated with each adverse event is derived at the end of the analysis. A computer program has been developed to harness high computation power required for the Bayesian approach (*See* **Example 12).** The program provides an interactive platform to visualize the outcome of Bayesian estimation approach with user-provided spreadsheets. The program also provides parameter tuning modules to define initial priors, remove outliers, and select multiple attributes. A few sets of real clinical data from previous CIA performed for mAb D as well as a few modeled data based on the mAb D CIA data were tested on the system. The Bayesian estimation method generated expected results, demonstrating the validity of the system.

## EXAMPLES

### EXAMPLE 1: Methods of molecular attribute analysis

Here, we examine the clinically relevant immunological safety impact by 15 molecular attributes between 2 immunoglobulin G2 (IgG2) monoclonal antibody (mAb) drug products, mAb A and mAb B, using a data-analysis approach, dubbed Clinical Impact Analysis (CIA). CIA tests whether a correlation exists between the level of patient exposure to a given attribute and the extent of the occurrence of a clinical outcome by analyzing the data from clinical studies and product quality analysis studies. No association was found between the level of the patient exposures to any of the attributes and the ADA response, suggesting that that none of analyzed attributes at the examined exposure levels causes ADA response. Moreover, upper ranges of the examined exposure levels were higher than, or near, the purity specification limits for the attributes of mAb A, demonstrating the feasibility of generating clinically relevant evidence for justifying these attributes made possible by CIA. These results show that integrating clinical and analytical information provides clinically relevant insights into attribute impact applicable in drug development and manufacturing.

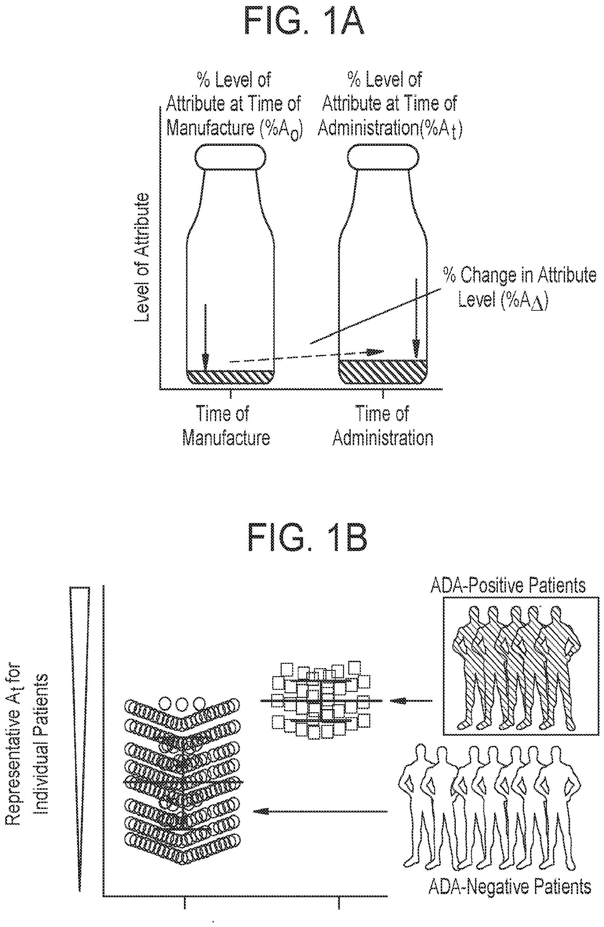

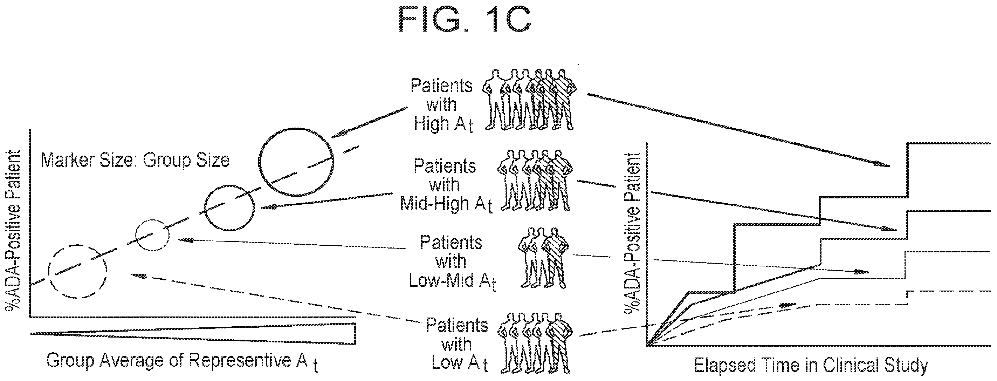

We assess clinically relevant safety impact of attributes by integrating the clinical and attribute analytical data (Figure 1, Equation 1). Briefly, the attribute exposure level at the time of treatment administration is approximated by combining the attribute level measured at lot release with the change in attribute level between lot release and the treatment administration time (Figure 1A). Attribute exposure is then determined for individual treatments administered to the patient during the treatment regimen. If an attribute causes a clinical event, then the patients with the event will be found associated with higher levels of exposure to the attribute on average (Figure 1B), or a positive correlation will be found between the attribute exposure level and the incidence (Figure 1C, left) or time course (Figure 1C, right) of the clinical event. Conversely, absence of the association between the attribute exposure and clinical event will indicate that the attribute does not cause the event at the examined exposure levels.

As shown in **FIG 1A,** attribute-exposure level at the treatment administration time, $A_t$, is approximated by accounting for the attribute level measured at the time of manufacture, and change in the level over time. In a retrospective analysis, patients are grouped by the retrospectively known clinical outcome, such as ADA test results **(FIG 1B).** Then, $A_t$ representing individual patients are compared between the groups as illustrated. In a prospective analysis, patients are grouped according to the representative $A_t$ **(FIG. 1C).** The incidence (left) or time course (right) of clinical outcome for each group is compared with respect to the group's average representative $A_t$.

We investigated the immunological response of patients exposed to 15 attributes between 2 separate drug products, mAb A and mAb B, by examining the attribute exposures and ADA test results for 1,398 patients from 8 individual clinical studies for mAb A, and for 5,439 patients from 2 combined clinical studies for mAb B. ADA remains a critical regulatory concern due to a range of impacts on safety and efficacy, including neutralization of the target-binding function by the drug and other more serious adverse events[17,18].

The attributes analyzed for mAb A are high molecular weight (HMW) species in size exclusion chromatography (SEC), acidic species in cation exchange (CEX), non-heavy-chain and light-chain (NonHC+LC) species in capillary electrophoresis in reducing/denaturing condition (rCE-SDS), and hydrophilic species in the pre-peak that elutes before the main species in hydrophobic interaction chromatography (HIC). The attributes analyzed for mAb B are SEC HMW; rCE-SDS-separable low molecular weight (LMW) species, mid-molecular weight (MMW) species, non-glycosylated heavy chain (NGHC) species, sum of LMW MMW and NGHC (L+M+NG), and NonHC+LC; high-mannose (HM) species analyzed by hydrophilic interaction chromatography, CEX-separable basic species 3 and all basic species; and C-term sequence variant 1 (CSV1) and C-term sequence variant 2 (CSV2) analyzed by mass spectrometry. It is contemplated that for methods described herein, any attribute or combination of attributes can be analyzed.

At the attribute exposure levels examined, we find that none of the 4 attributes of mAb A is a determinant of ADA. Also none of the 11 attributes of mAb B is found to be a determinant of ADA. Moreover, the upper ranges of the examined levels of attribute exposures are higher than, or near, the proposed purity specifications for the corresponding attributes of mAb A, demonstrating that CIA can be used in generating clinically relevant evidence justifying the proposed specifications.

CIA entails analyzing the extents of prevalence, or time course, of clinical outcome per a given variable, such as varying levels of attributes exposed to patients. The following approaches were carried out to integrate the ADA test results and attribute-exposure level for individual patients in performing CIA.

*Clinical Study Selection*

Criteria for selecting clinical studies for CIA were that (1) a large variability in the attribute-exposure level was present, and (2) a large number of patients was enrolled. This way, statistically meaningful results can be obtained from analyzing the clinical impact in the context of wide range of attribute-exposure levels. So, dose-escalation studies, or studies that used multiple treatment lots, were selected, and, if larger patient number was needed, patients from comparable studies were combined for the analysis.

*ADA Test Results from the Clinical Studies*

The ADA test results from electrochemiluminescence (ECL)-based bridging immunoassays[19], each developed to specifically detect the mAb A- or mAb B-binding ADA in the patient serum, were parsed for the analysis. It is noted that binding ADA encompasses both neutralizing and non-neutralizing ADAs. However, neutralizing ADAs are far less prevalent than binding ADAs for both mAb A and mAb B, requiring a far larger patient number for statistically meaningful analyses, and analyzing the binding ADAs fits the present Example, which tests whether any clinical outcome is associated with a variable in treatments.

The clinical studies selected for mAb A were 4 early-phase dose-escalation studies with up to total 218 patients, 2 Phase 2 studies with up to 627 patients, and 2 Phase 3 studies with up to 542 patients. These 3 sets of studies were individually analyzed by retrospective or prospective CIA described below.

The clinical studies selected for mAb B were 2 Phase 2 studies with up to 5,440 patients. This set of studies were analyzed by both retrospective and prospective CIA.

*Attribute Analysis and Stability Study Data*

The drug-product batches administered in the clinical studies selected for CIA were traced using batch trace record to identify the drug-product lots inspected in product quality analytical tests, so the attribute level determined at the time of lot release specific to individual treatment lots could be obtained.

The tests for lot-release analyses are SEC (size-exclusion chromatography) for HMW, rCE-SDS (capillary electrophoresis in reducing, and SDS-denaturing condition) for HC, LC, LMW, MMW and NGHC, and CEX (cation-exchange chromatography) for acidic or basic species, individually developed specifically for mAb A or mAb B. Other analytical data from HIC (hydrophobic-interaction chromatography), Hydrophilic chromatography (HILIC) for HM, and peptide-mapping by LCMSMS (liquid chromatography-tandem mass spectrometry) for C-terminal sequence variant species (CSV1 and CSV2) were available for limited batches of mAb A or mAb B because they were not in the panel of lot-release assays.

The data from attribute stability studies were also mined for each drug product. The stability data represent the change in the level of individual attributes over time in the storage condition, critical to determining the attribute-exposure level at the time of treatment administration as below.

*Determination of Attribute Exposure*

The amount of attribute exposure at the time of treatment administration, $A_t$, associated with each treatment was determined from the information above using Equation 1:

$$A_t = (\%A_0 + \%A_\Delta \times t)D \qquad\qquad \text{(Equation 1)}$$

where $\%A_0$ is the percentage of attribute at the time of lot release, $\%A_\Delta$ is rate of change in the percentage of attribute level, t is the storage time between lot release and treatment administration, and D is the dose strength in the dimension of weight of active pharmaceutical ingredient associated with each treatment.

Each patient is then assigned with a representative $A_t$. For early-phase studies where single treatment is administered to each patient, the representative $A_t$ was $A_t$ associated with the treatment.

For studies involving multiple-treatment regimens, the treatments administered after the first time that the patient is tested positive for ADA are irrelevant to investigating the cause of ADA. So, for the ADA-positive patient, the representative $A_t$ was the average, or maximum, of the attribute-exposure levels associated with the treatments administered prior to the first time that ADA was tested positive. For the ADA-negative patient, all treatments in the entire regimen were included in determining the average or maximum exposure to obtain the representative $A_t$.

*Patient Groups for CIA*

Of the patients from the studies selected as described above, only the ones meeting the following criteria were included in CIA: (1) Batch information is available for all treatments in the regime; (2) the attribute exposure could be determined for all treatments in the regime; (3) the ADA-test results are available; and (4) no ADA test conducted prior to administering the first treatment yielded positive results. For instance, one patient from the clinical study for mAb B was excluded from CIA because an entry for the drug product lot number was missing for one of the treatments. Also, 29 patients in the studies for mAb B were excluded because they were tested positive for ADA prior to the treatment.

Applying these criteria resulted in the following number of patients: CIA for HMW, NonHC+LC or acidic species of mAb A separately analyzed 218 patients from the 4 combined early-phase studies, and 627 patients from the 2 combined Phase 2 studies. CIA for HIC Pre-Peak of mAb A separately analyzed 90 patients from the 2 combined early-phase studies, and 24 patients from the 2 combined Phase 2 studies. A separate CIA for HMW of mAb A additionally analyzed 553 patients from the 2 combined Phase 3.

For HMW, LMW, MMW, NGHC, M+N+NG, NonHC+LC, basic species 3 and all basic species of mAb B, 5,439 patients from the 2 combined Phase 2 studies were analyzed by all 3 CIA approaches (Figure 1B and C, see below). CIA for HM of mAb B was performed by analyzing 127 patients, and for CSV1 and CSV2, by analyzing 1,671 patients, from the same set of clinical studies.

## Example 2: Results for analysis of attribute impact for individual patients

If an attribute causes an ADA response, then a positive correlation would exist, so the ADA-positive patients would be found associated with higher attribute-exposure level as compared to the ADA-negative patients **(FIGs. 1A-C).**

Finding the association does not prove the causation, although such finding can prompt further studies to test the causation. However, discovering that such association is absent will eliminate the causation of ADA by the attribute at the examined exposure level. Three separate analyses were performed to test the association between the attribute-exposure level and the occurrence of ADA response.

The representative attribute-exposure level analyzed for individual patients (as described in **Example 1)** was determined from the single exposures for the patients in the Phase 1 studies for mAb A, maximum exposures for the patients in the Phase 2 and 3 studies for mAb B, and maximum exposures for the patients of mAb B. Average $A_t$ is a statistical representation of the multiple attribute exposures. Maximum $A_t$ is immunologically relevant because the patient may generate ADA only upon an exposure to an immunogen exceeding a certain threshold level[20,21].

## Example 3: Retrospective CIA for Attributes of mAb A and mAb B

Retrospective analysis (subjects grouped by the retrospectively known clinical outcome) was developed to test whether the ADA-positive patients were associated with higher attribute-exposure levels as compared to the ADA-negative patients. Patients are grouped according to retrospectively known ADA test results **(FIG. 1B).** Then, the distributions of the representative attribute-exposure levels are compared between the ADA-positive and ADA-negative patient groups for each attribute. If the representative attribute-exposure levels for ADA-positive patient group are higher than those for the ADA-negative patient group on an average, then Student's t-test is performed to assess whether the difference is statistically significant.

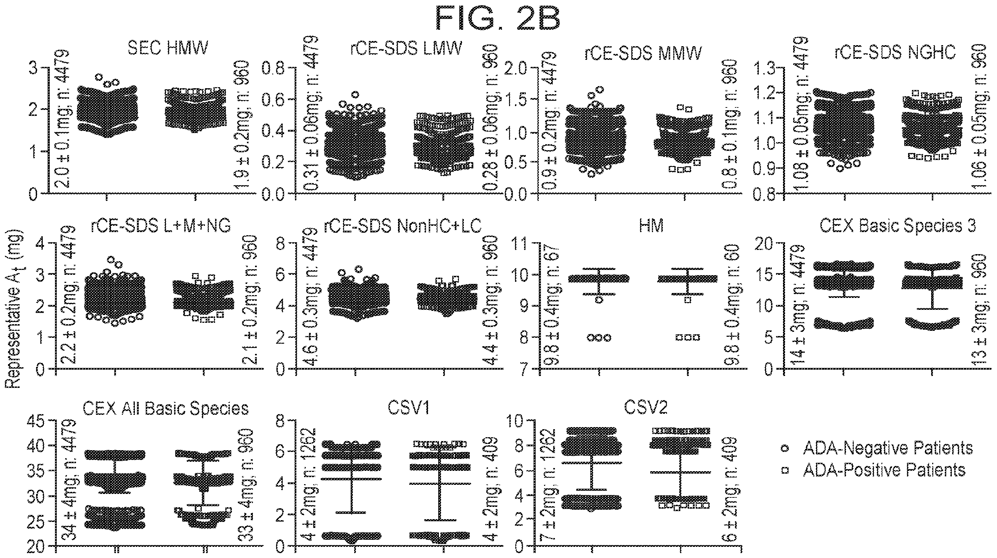

Association is found statistically absent between the ADA-positive patients and higher exposure to any of the attributes of mAb A or mAb B **(FIG. 2),** indicating that none of these attributes causes ADA at the examined exposure levels. This finding stands regardless of whether the single- **(FIG. 2A),** average- (Appendix Figure 1), or maximum-exposure **(FIG. 2B)** was used as the representative exposure level (See Methods section). Examining multiple sets of clinical studies for mAb A at various phases synonymously indicates that none of the 4 attributes is a determinant of ADA **(FIG. 2A, FIGs. 7A** and **8).**

**FIGs. 2A-B** illustrate comparisons of the levels of attribute exposures between the ADA-positive and ADA-negative patients show statistically significant association is absent between the high exposure level and ADA-positive patients for all analyzed attributes, indicating that these attributes do not cause ADA in the examined clinical conditions. Numbers in parentheses are p-values determined when needed. The lines are the average $\pm$ standard deviation of individual patients' representative attribute exposures for each group.

## Example 4: Prospective CIA (of ADA Incidence for Attributes of mAb A and mAb B

Incidence analysis in subjects grouped according to the representative $A_t$ was developed to test whether a positive correlation exists between the attribute-exposure level and the incidence of ADA response. The patients are grouped in quartiles according to the attribute-exposure levels, and the % ADA-positive patient determined for each group is compared with each other with respect to the group average of the representative attribute-exposure levels **(FIG. 1C,** Left Panel).

The ranges of the exposure levels applied to create bins for the quartiles were optimized to achieve minimal variabilities in exposure levels within each patient group, and simultaneously to ensure adequate number of patients constituting each group. If a positive correlation was observed between the incidence of ADA and attribute-exposure level, a weighted correlation was computed using the patient number in individual groups as the weight[22] to test whether the correlation was statistically significant.

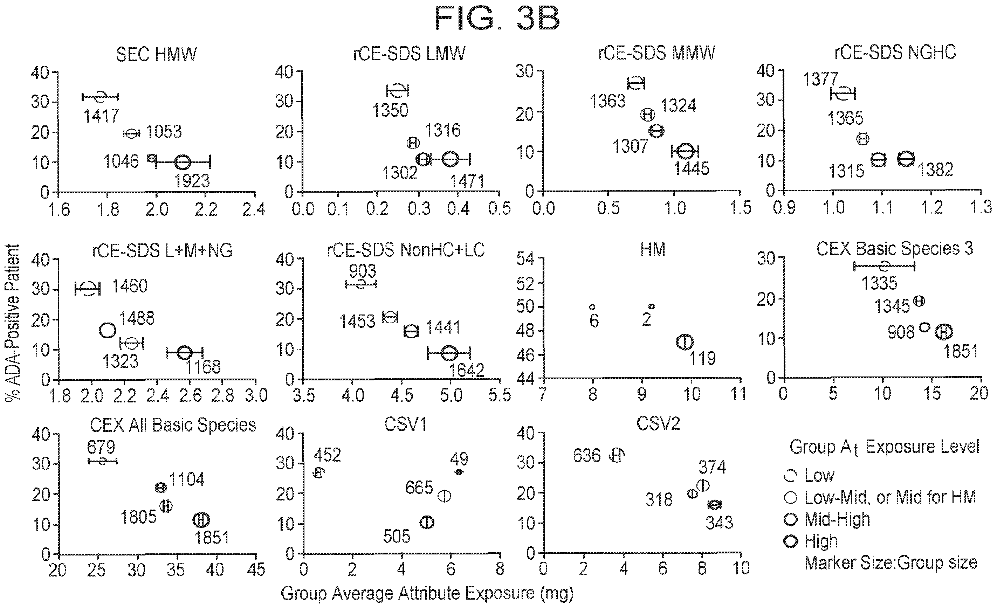

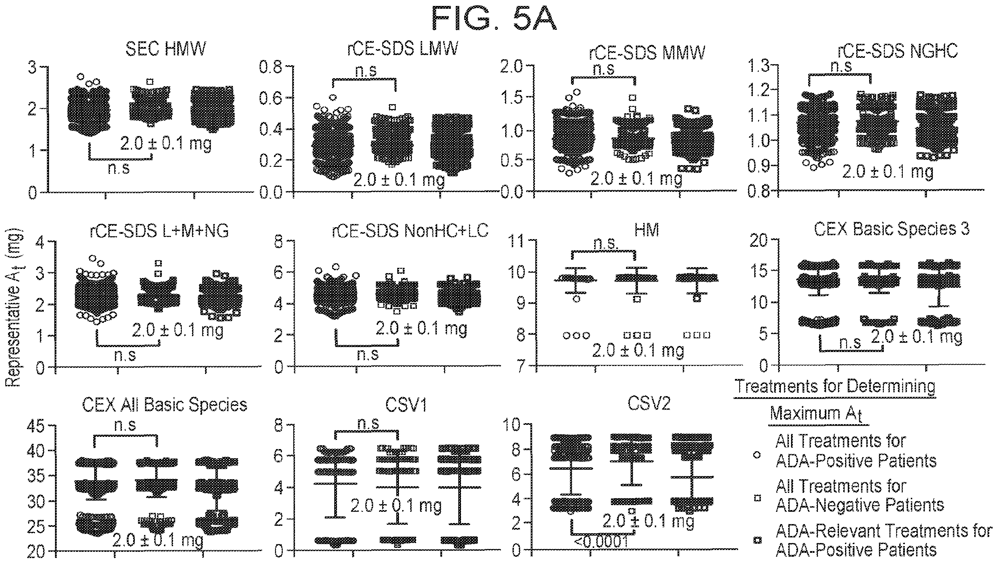

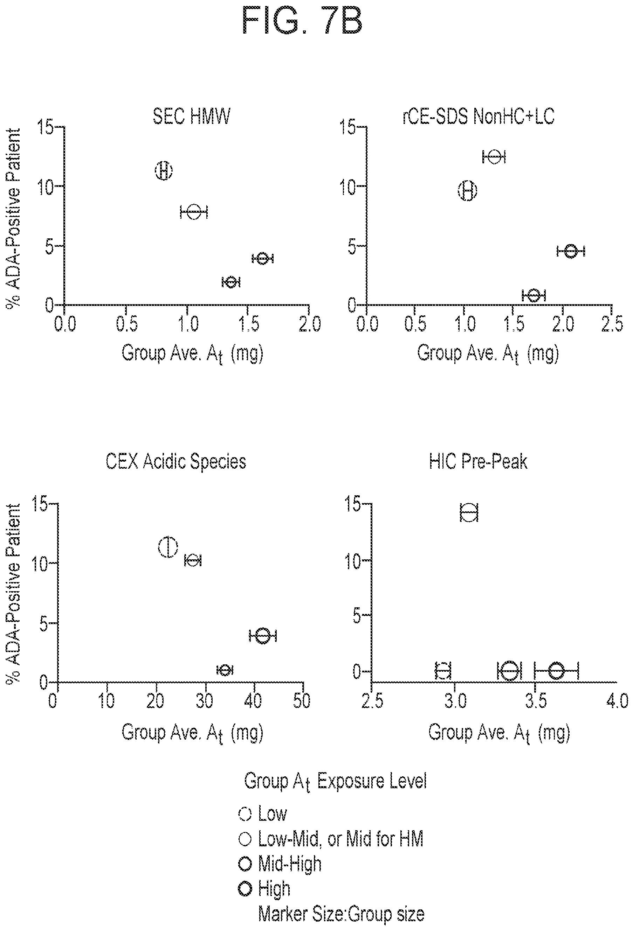

Consistent with the results from the CIA analysis of **Example 3** (in which subjects were grouped by the retrospectively known clinical outcome), no correlation is found statistically positive between the incidence of ADA and the exposure level for all attributes analyzed for mAb A or mAb B **(FIGs. 3A-B, FIG. 7B).** In fact, lower exposure appears associated with ADA-positive patients of mAb B, manifested as negative correlations in the Incidence for subjects grouped according to the representative $A_t$ **(FIG. 3B).** This negative correlation is a statistically informative outcome that additionally corroborates the absence of causation (See Below; **FIGs. 5A-B, FIG. 9).**

**FIGs. 3A-B** show fractions of ADA-positive patients in the patient quartiles grouped according to the representative attribute exposure levels ranging from low to high are plotted as a function of the group average of the representative exposure levels. Numbers in the parentheses are weighted p-values determined when a positive correlation is found. The number of patients in each quartile is next to the plot. Error bars are standard deviations. Results for mAb A are shown in **FIG. 3A,** and results for mAb B are shown in **FIG. 3B.**

## Example 5: Prospective CIA analysis of ADA Time Course for Attributes of mAb A and mAb B

Another prospective analysis was developed (subjects grouped according to the representative $A_t$; *See* **FIG. 1B,**

right panel) to compare the time course of ADA response among the patients grouped in the same way as in the CIA Incidence in Example 4, above. If higher attribute-exposure level was associated with more extensive occurrence over time, then Mantel-Cox log-rank test23,24 was performed to test whether the difference in the time courses was statistically significant.

Consistent with above analyses, no association was found between the time courses of ADA and exposure levels for all attributes of mAb A or mAb B **(FIGs. 4A-B).** For HIC Pre-Peak of mAb A **(FIG. 4A),** the result showing the absence of its association with ADA was non-informative due to the limited number of patients analyzed. Other analyses above performed using data for a larger number of patients in a different set of clinical studies **(FIGs. 2A and 3A)** indicate that HIC Pre-Peak, indeed, is not a determinant of ADA.

## Example 6: Association between ADA Response and Lower Representative $A_t$

The association between lower $A_t$ and the ADA response, or negative correlation, is found in both incidence and time course for both mAb A and mAb B **(FIGs. 2B, 3B, 4A and 4B).** This negative correlation becomes pronounced when analyzing maximum $A_t$ in CIA for mAb B **(FIGs. 2B, 3B and 4B).**

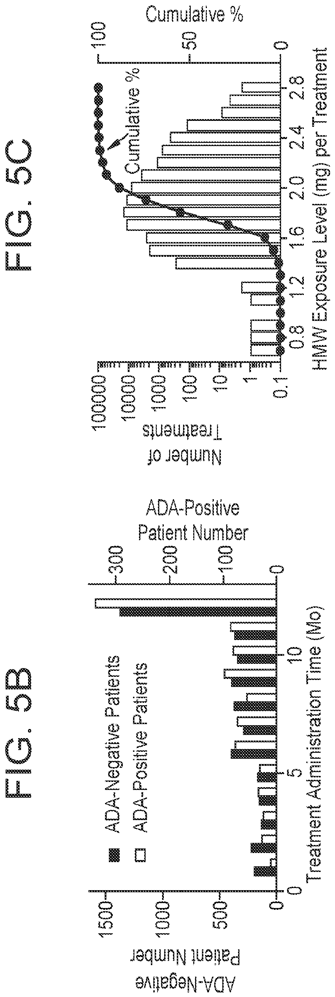

Using HMW exposures in the mAb B-treated patients, we show that the negative correlations strengthen the results from CIA showing that the attribute exposure does not cause the ADA response. No bias exists in how maximum HMW exposure occurs between the ADA-negative and ADA-positive patients: There is generally no difference in the level of maximum $A_t$ between ADA-negative and ADA-positive patients (Figure 5A); and, the treatment times, at which maximum HMW exposures occur, are also similarly distributed between the ADA-negative and ADA-positive patients (Figure 5B). However, most treatments are associated with HMW exposures that are, particularly, lower than the average of maximum HMW exposures of 2 mg as in Figure 5A (Figure 5C). Without being limited by theory, it is contemplated that excluding the treatments at some of the later administration times (due to their lack of relevance to the causality for the occurrence of ADA) may result in the analysis of the next highest HMW exposures as representative exposures for the ADA-positive patients. The use of these next-highest HMW exposures may create a bias to nudge the correlation in a negative direction. This potential bias may be taken into account when performing the analysis.

In **FIG. 5A,** the maximum $A_t$ was determined using all treatments or ADA-relevant treatments are plotted for the ADA-negative or ADA-positive patients. Average $\pm$ standard deviation for maximum $A_t$ determined using all treatments for ADA-negative patients are indicated; and those for other groups are as analyzed for the same corresponding groups in Figure 2B. p-values from the t-tests are higher than 0.05 for the comparisons indicated n.s. (not significant), unless indicated otherwise. The error bars are standard deviation. In **FIG. 5B,** frequencies of the time for monthly treatments linked to the maximum HMW exposures in individual patients are shown for the ADA-negative and ADA-positive groups. In **FIG.** 5C, frequency of treatments resulting in different levels of HMW exposures for individual mAb2 patients is plotted, along with the cumulative percentage of the treatment numbers.

Only the attribute exposures from the treatments administered prior to the ADA responses are relevant to ADA response. Also, analyzing the maximum attribute exposures takes immunological mechanism into consideration, as ADA may only be induced by immunogens exceeding threshold concentrations20,21. Taken together, the negative correlations found here provide added evidence that the attribute exposure does not cause ADA (See **Example 8).**

## Example 7: Applying the Approach of CIA in Analyzing the Clinical Impact of Treatment Regimen

In addition to analyzing the attribute impact, we tested whether an association exists between the ADA response and various factors of the treatment regimen, including the number of treatment administrations, dose strengths for each treatment, and number of treatment lots associated with the course of treatment for each patient.

None of these factors is found associated with the incidence of ADA. Indeed, the ADA-positive patients are associated with lower numbers and durations of treatments, as well as fewer treatment lots, because the analyses included only the treatments given before their first positive ADA tests.

This demonstrates that the approach of CIA can also be applied to other aspect of treatments, in addition to attribute exposures. We envision that powerful analysis that expands the knowledge in drug safety and efficacy can be performed by combining the attribute exposure information to the patient information, such as that for demography, or even genotypes.

## Example 8: An additional analysis of prospective CIA incidence

In CIA Incidence for subjects grouped according to the representative $A_t$ **(FIGs. 9A-B),** whether the slope of the linear regression is significantly different from zero was tested by using F-test, which calculates the probability, p, that randomly selected points would result in, or exceed, the observed linear-regression correlation coefficient, $R^2$. The p-values from the F-tests that determined the significant non-zero slopes in the regressions for rCE-SDS NonHC+LC, CEX Basic Species 3, CEX All Basic Species and CSV2, analyzed using max $A_t$, were 0.017, 0.049, 0.031, and 0.045, respectively. Other slopes were not significantly different from zero.

**FIG. 9B** is recapitulated, and compared to the same sets data that are re-analyzed using average $A_t$, instead of maximum $A_t$ as FIG. 9B. Lines are non-weighted linear regression, and the slope of all regressions is statistically zero, except for those for rCE-SDS NonHC+LC analyzed using maximum At, CEX Basic Species 3 analyzed using maximum At, CEX All Basic Species analyzed using maximum $A_t$, and CSV2 analyzed using maximum $A_t$.* Number of patients binned in the quartiles for re-analyses is same as the corresponding quartiles as in FIG. 9B, unless indicated otherwise. The correlations for the re-analyzed data are generally less negative, except for HM and CSV1, the slope for all fits of which are statistically zero.

## Example 9: CIA using Additional Clinical Studies

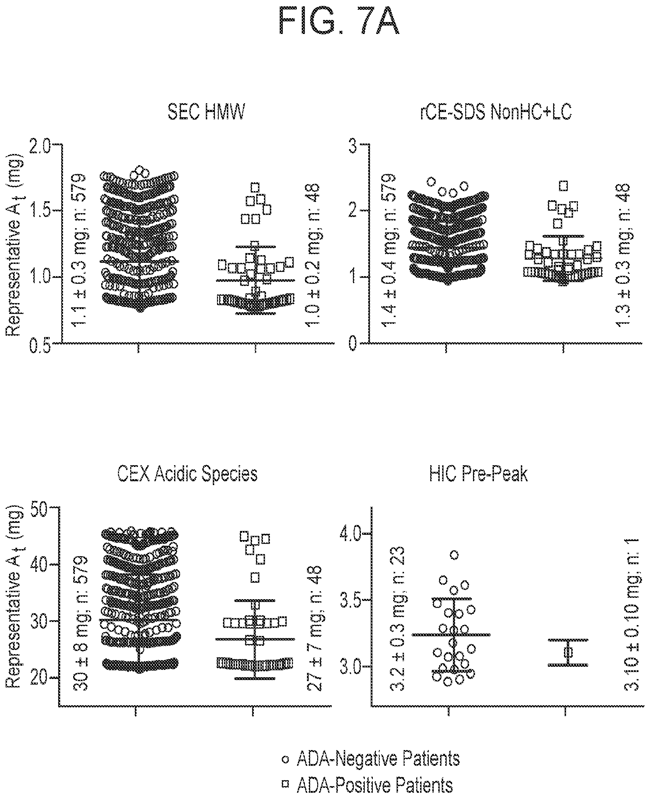

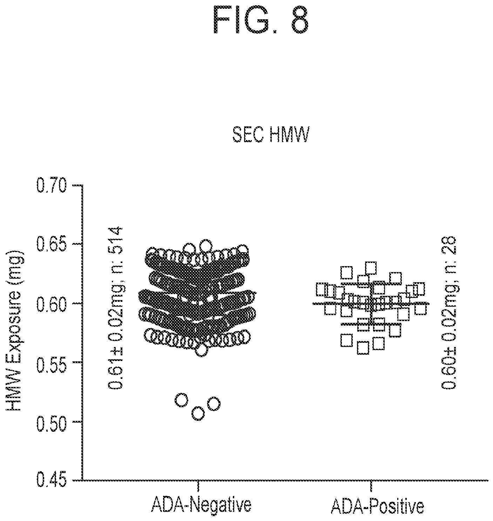

The CIA analyses for impact of attributes of mAb A on ADA performed using the data from Phase 2 clinical studies indicate that none of the CQAs is a determinant of ADA response at the examined exposure levels **(FIG. 7).** The attribute-exposure levels in the Phase 3 clinical studies of mAb A are lower than those in the earlier phase studies, and, as indicated by the CIA analysis of subjects grouped according to the representative $A_t$, do not cause ADA **(FIG. 8).**

As shown in **FIG. 7-B,** patients on regimen in clinical studies different from those analyzed in **FIG. 2A** are analyzed. Average $A_t$ per a treatment is used as representative $A_t$ for individual treatments. Error bars are uncertainty propagated from standard deviation associated with individual average $A_t$. The quartiles analyzed in **(FIG. 7B),** and the attribute exposure ranges used to group the quartiles, are same as those in **FIG. 4A.**

*Association between Low $A_t$ and ADA*

A bias will be found in maximum attribute exposures between the ADA-negative and ADA-positive patients, if the attributes do not cause ADA and only the ADA-relevant treatments are analyzed in the CIA of ADA-positive patients, as established herein. Such bias will decrease when the average exposure per a treatment is analyzed as the representative exposure for individual patients. For instance, plausibly, the bias resulting in lowered maximum HMW exposures upon excluding ADA-irrelevant treatments from the CIA of ADA-positive patients may be offset by the analogous bias resulting in heightened minimum HMW exposures. Consistent with this illustration, using the average exposures as the representative exposures for CIA generally diminishes, or eliminates, these negative correlations (FIG. 9). Because $A_t$ analyzed here are relevant to ADA, and analyzing maximum exposures is immunologically significant 25,26, negative correlations described in the main text provide added evidence that the attributes do not cause ADA in the examined clinical condition.

## Example 10: Analysis and Conclusions for Examples 1-9

We show that integrating the information on treatment lots, clinical outcomes and attribute analytics data enables the approximation of the attribute-exposure level associated with individual treatments administered to the patients. Then, whether an attribute causes ADA can be tested by statistical tests that examines the presence of correlation between higher $A_t$ and occurrence of ADA response. While the presence of association will require further focused investigations to establish the causation, absence of association eliminates causation.

The work here describes an effort that integrates treatment info for individual patients and assesses the clinically relevant impact of molecular attributes for therapeutics. Here, no significant positive correlation is found between the ADA response and exposure level for any of 15 attributes examined between 2 drug products, mAb A and mAb B, indicating that none of these attributes is a determinant of ADA at the examined levels for the corresponding drug products **(Table 1).** This work does not establish that these attributes also pose no increased safety risk in different exposure levels or for other therapeutic mAb molecules.

The present approach analyzes passive data mined from clinical studies originally intended to focus on establishing safety or efficacy of the drug products. Ethical concerns in exposing patients to arbitrary levels of attributes preclude generation of active data by designing studies specifically to explore the attribute impact in patients. Nevertheless, CIA of passive data provides rich insights and implications for both applied and basic scientific pursuits in biotech.

For instance, CIA can help to determine the patient-centric product purity specification limits. Lack of causation of ADA by attribute exposures, as established above, indicates that no increased risk of immunological safety is posed by the attributes at the examined exposure levels. Notably, the upper limits of these attribute-exposures levels analyzed for mAb A **(Table 1)** are near, or higher than, the limits on corresponding attributes imposed by the specifications of this drug product (not shown), demonstrating that patient-focused evidence generated by CIA can be used for determining the therapeutics specification limits.

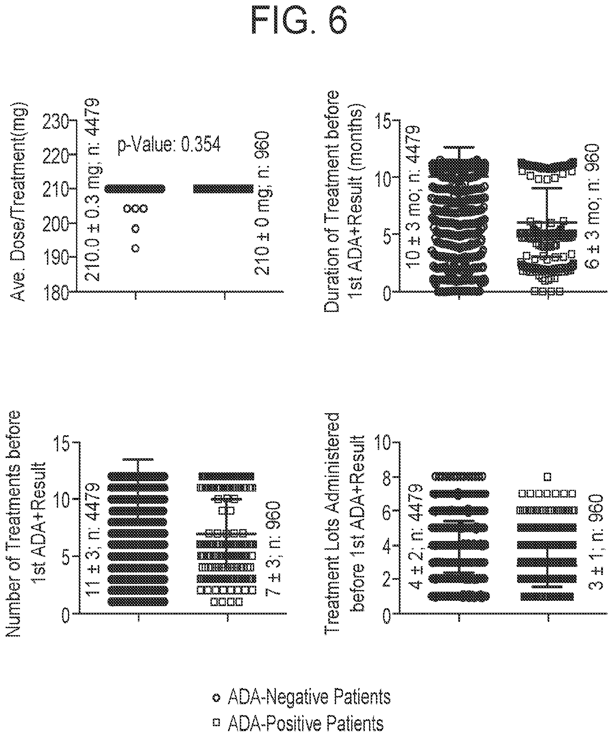

**FIG. 6** illustrates impacts of factors in treatments on ADA. Various factors of mAb B treatment regimen, average dose strength per a treatment, treatment duration, treatment number and treatment lot number are compared between ADA-negative and ADA-positive patients. These factors are of all treatments for ADA-negative patients, or of treatments prior to the first ADA-positive test results for ADA-positive patients. Errors are standard deviation.

**Table 1. Average and Maximum of Representative Attribute Exposures (in mg) Analyzed of Individual Patients in CIA.**

| Molecule | Attribute | Average | ± | Standard Deviation | Maximum |
|---|---|---|---|---|---|
| mAb A[†] | SE HMW | 1.0 | ± | 0.5 | 2.8 |
| | CE NonHC+LC | 1.7 | ± | 0.9 | 4.4 |
| | Acidic Species | 36 | ± | 17 | 87 |
| | HIC Pre-Peak | 4.9 | ± | 0.4 | 5.2 |
| mAb B[‡] | SE HMW | 2.0 | ± | 0.2 | 2.8 |
| | CE LMW | 0.30 | ± | 0.06 | 0.63 |
| | CE MMW | 0.9 | ± | 0.2 | 1.7 |
| | CE NGHC | 1.08 | ± | 0.05 | 1.20 |
| | CE L+M+NG | 2.2 | ± | 0.2 | 3.5 |
| | CE NonHC+LC | 4.6 | ± | 0.3 | 6.3 |
| | HM | 9.8 | ± | 0.4 | 9.9 |
| | Basic Species 3 | 14 | ± | 3 | 17 |
| | All Basic Species | 34 | ± | 4 | 39 |
| | CSV1 | 4 | ± | 2 | 6 |
| | CSV2 | 6 | ± | 2 | 9 |

[†]Values for mAb A are from **FIGs. 2A** and **3A.**
[‡]Values for mAb B are from **FIGs. 2B, 3B** and **4B.**

CIA can also deepen fundamental knowledge in clinical immunology. Any association found between the ADA response and attribute exposure level can prompt focused *in vitro* or *in vivo* studies to investigate the causation or mechanism of ADA generation. Depending on whether average or maximum $A_t$ better represents any attribute exposure found correlated to ADA, a hypothesis can also be narrowed to test whether the repeated high attribute exposures or single high attribute exposures are critical to inducing ADA response.

We further show that CIA can be combined with other analyses to provide powerful implications in therapeutics development, and to address uncharted critical questions. Can an attribute impact the clinical outcome of a subset of population defined by their demographic or genomic information? Could resulting knowledge in demography and genomics advance clinical trial design? Would attributes alter biophysical behaviors, such as structure and conformational dynamics, for different therapeutic protein molecules, and in turn, impact clinical outcomes? The present work reports data and analyses that spearhead these and further discussions.

In summary, understanding the impact of molecular attributes on drug safety and efficacy is critical to therapeutics development. For instance, some attributes may cause patients to generate anti-drug antibody (ADA) responses, which remains an important regulatory concern due to the potential clinical impacts, including neutralization of the drug. Non-human model systems, such as cells, tissues and animals, are viable experimental systems for testing safety impact of the attributes, but they provide limited insights into the clinical consequences of patient exposure to attributes. Here, we examine the clinically relevant immunological safety impact by 15 attributes between 2 immunoglobulin G2 (IgG2) monoclonal antibody (mAb) drug products, mAb A and mAb B, using a data-analysis approach, dubbed Clinical Impact Analysis (CIA). CIA tests whether a correlation exists between the level of patient exposure to a given attribute and the extent of the occurrence of a clinical outcome by analyzing the data from clinical studies and product quality analysis studies. No association was found between the level of the patient exposures to any of the attributes and the ADA response, suggesting that that none of analyzed attributes at the examined exposure levels causes ADA response. Moreover, upper ranges of the examined exposure levels were higher than, or near, the purity specification limits for the attributes of mAb A, demonstrating the feasibility of generating clinically relevant evidence for justifying these attributes made possible by CIA. These results show that integrating clinical and analytical information provides clinically relevant insights into attribute impact applicable in drug development.

## Example 11: Analysis of Product C, a canonical BiTE® molecule

A further analysis was performed on Product C, a canonical BiTE® molecule. The molecular attributes measured were Tyr Sulfation, Asp Isomerization, Lys Hydroxylysine, Lys Glucosyl-galactosyl Hydroxylsine, Met Oxidation, and SE-HPLC Aggregates. Potency was also assessed. Safety data included identification of the following adverse events: Anemia, Cytokine Release Syndrome (CRS), Fever, Infusion Related Reaction (IRR), Lymphocytopenia, and Neurological Events.

Attribute exposure was estimated based on 1) the % level of attribute for different drug-product lots at the time of manufacturing, 2) the attribute stability, or the rate at which the level of attribute changes over the duration of DP storage, 3) the date and time at which an individual treatment is administered to each patient, along with the record of which drug-product lot was administered for each treatment. Then, 4) the date and time of the occurrence of the given clinical outcome with the grades (or severities) of interest can be incorporated for the next step in CIA (Figure 1), where 5) CIA combines the information to determine the attribute exposure, and 6) analyzes whether an association exists between the level of attribute exposure and the occurrence of the clinical outcome of interest.

(A) $A_t$ was first determined using Equation 1 for all relevant treatments as defined below.

(1) For patients who do not show the clinical outcome of interest throughout the course of the clinical studies, the relevant treatments are all treatments administered to the patients throughout the study.
(2) For patients showing the clinical outcome, the relevant treatments are all treatments administered to the patients prior to the occurrence of the clinical outcome.
(3) For patients who develop the clinical outcome prior to receiving any treatment, no relevant treatment exists. $A_t$ was 0 for these patients.
(4) For administrations via continuous infusion, $A_t$ that occurs during the relevant portion of the infusion was determined as below:

a. For infusion continuously administered for longer than 24 hours, $A_t$ per each 24-hour-long segment of the relevant infusion prior to occurrence of the clinical outcome is determined.
b. For infusions that are less than 24 hours long, $A_t$ associated with each of the relevant infusion prior to the occurrence of the clinical outcome is determined.
c. For either of the above 2 cases, if the clinical outcome occurs during an infusion, $A_t$ associated with the portion of the infusion prior to the occurrence of the clinical outcome is determined.

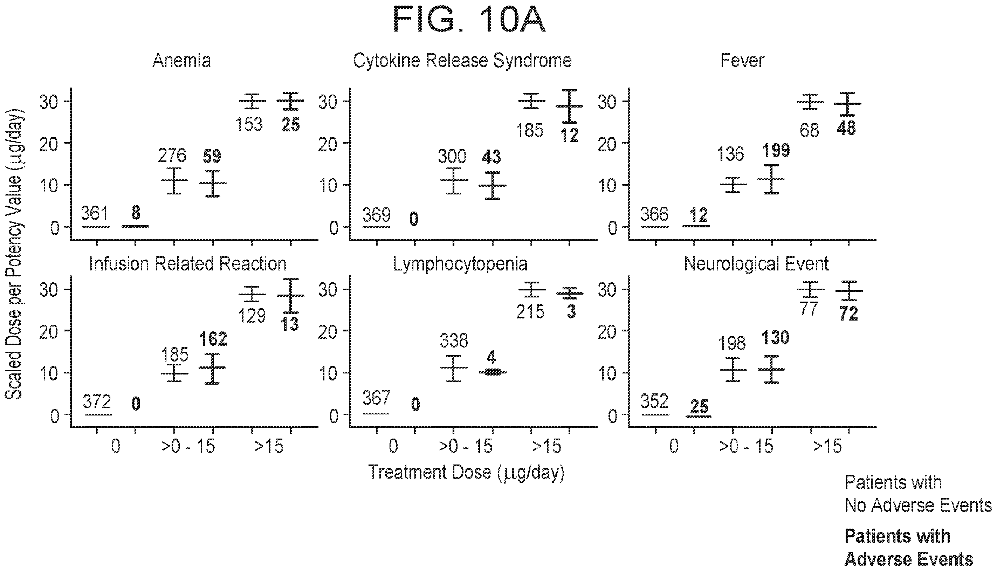

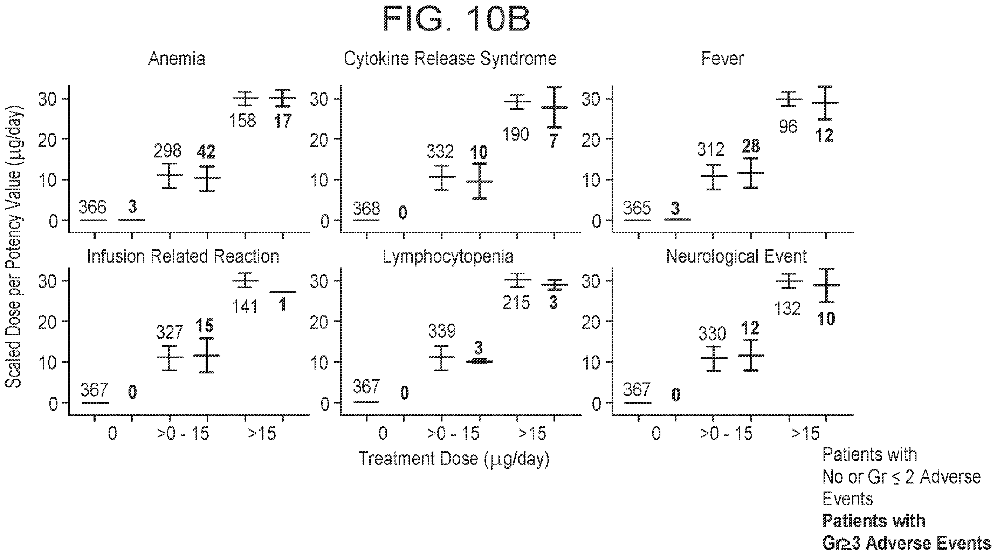

The results for the analysis of Product C are shown in FIGs. 10A-B. In addition to the 6 CQAs listed for CIA of Product C, the impact of potency on the AEs was analyzed by testing whether a correlation exists between the Product C dose scaled per the potency value and the occurrence of the AEs for patients administered with no dose, mid dose or high dose levels. As shown in FIG 10A, association of potency with the occurrence of AEs with any grade was analyzed. As shown in Fig. 10B, association of potency with the occurrence of AEs with a grade of 3 or higher was analyzed.
Is was observed that none of the attributes were correlated with any of thereof adverse events tested. Thus it was determined that the estimated levels of attribute exposure were safe.

**Example 12: Computer implementation of Bayesian estimation**

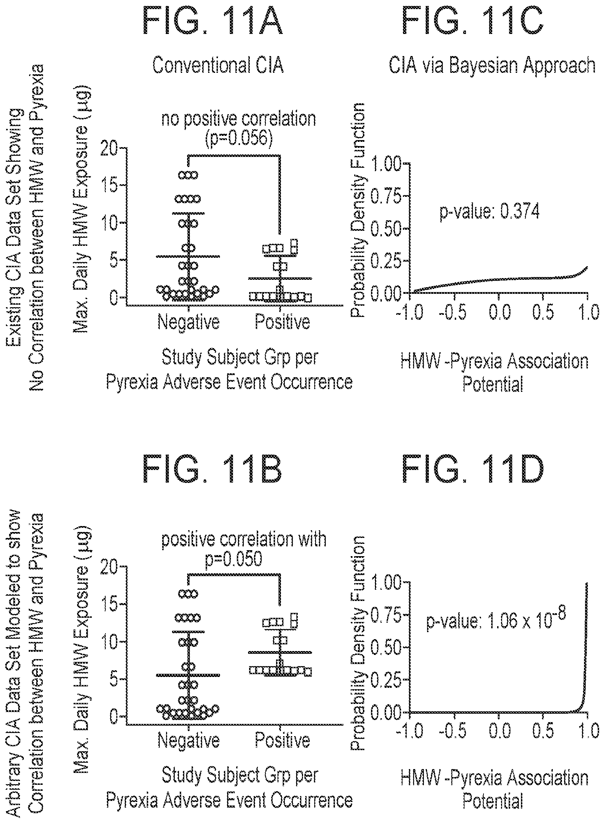

A computer program was developed to harness high computation power required for the Bayesian estimation approach. The program provides an interactive platform to visualize the outcome of Bayesian estimation approach with user-provided spreadsheets. The program also provides parameter tuning modules to define initial priors, remove outliers, and select multiple attributes. A set of processed clinical data for methods of assessing a clinical impact of a molecular attribute of mAb D as well as a few modeled data based on the mAb D CIA data were tested on the system. The method was performed to test whether positive correlation exists between mAb D HMW exposure and the occurrence of pyrexia adverse event by comparing the maximum daily HMW exposures between the adverse event negative- and positive-subjects. The method found no correlation using the conventional method **(FIG. 11A)**. The Bayesian method **(FIGs. 11C-D)** generated analogous results to the conventional system **(FIGs. 11A-B),** demonstrating the validity of the system:

(1) when analyzing the clinical data for a mAb D clinical trial, the Bayesian estimation method indicated absence of probability that the attributes were correlated to the clinical outcomes **(FIG. 11C).** When the data were processed using conventional statistics, the same result was indicated by the conventional statistics **(FIG. 11A),** while
(2) when analyzing the modeled data set (based on the said clinical data), but in which the attribute exposure levels were arbitrarily increased for the clinical study subjects the Bayesian estimation method indicated a probability that the attributes were correlated to the clinical outcomes**(FIG. 11D),** as did the conventional statistics **(FIG. 11B).** Thus, the conventional statistics and the Bayesian estimation method both indicated the correlation between the clinical outcome and the arbitrarily increased attribute exposure levels.

### References

1 Rosenberg, A. S., Verthelyi, D. & Cherney, B. W. Managing uncertainty: a perspective on risk pertaining to product quality attributes as they bear on immunogenicity of therapeutic proteins. J Pharm Sci 101, 3560-3567, doi:10.1002/jps.23244 (2012).

2 Goetze, A. M., Schenauer, M. R. & Flynn, G. C. Assessing monoclonal antibody product quality attribute criticality through clinical studies. MAbs 2, 500-507 (2010).

3 Kelley, B., Cromwell, M. & Jerkins, J. Integration of QbD risk assessment tools and overall risk management. Biologicals 44, 341-351, doi:10.1016/j.biologicals.2016.06.001 (2016).

4 Bessa, J. et al. The immunogenicity of antibody aggregates in a novel transgenic mouse model. Pharm Res 32, 2344-2359, doi:10.1007/s11095-015-1627-0 (2015).

5 Bi, V. et al. Development of a human antibody tolerant mouse model to assess the immunogenicity risk due to aggregated biotherapeutics. J Pharm Sci 102, 3545-3555, doi:10.1002/jps.23663 (2013).

6 Bee, J. S., Goletz, T. J. & Ragheb, J. A. The future of protein particle characterization and understanding its potential to diminish the immunogenicity of biopharmaceuticals: a shared perspective. J Pharm Sci 101, 3580-3585, doi:10.1002/jps.23247 (2012).

7 Goetze, A. M., Liu, Y. D., Arroll, T., Chu, L. & Flynn, G. C. Rates and impact of human antibody glycation in vivo. Glycobiology 22, 221-234, doi:10.1093/glycob/cwr141 (2012).

8 Jawa, V. et al. Evaluating Immunogenicity Risk Due to Host Cell Protein Impurities in Antibody-Based Biotherapeutics. AAPS J 18, 1439-1452, doi:10.1208/s12248-016-9948-4 (2016).

9 Joubert, M. K. et al. Use of In Vitro Assays to Assess Immunogenicity Risk of Antibody-Based Biotherapeutics. PLoS One 11, e0159328, doi:10.1371/journal.pone.0159328 (2016).

10 Liu, Y. D. et al. Human IgG2 antibody disulfide rearrangement in vivo. J Biol Chem 283, 29266-29272, doi:10.1074/jbc.M804787200 (2008).

11 Liu, Y. D., van Enk, J. Z. & Flynn, G. C. Human antibody Fc deamidation in vivo. Biologicals 37, 313-322, doi:10.1016/j.biologicals.2009.06.001 (2009).

12 Yang, J., Goetze, A. M. & Flynn, G. C. Assessment of naturally occurring covalent and total dimer levels in human IgG1 and IgG2. Mol Immunol 58, 108-115, doi:10.1016/j.molimm.2013.11.011 (2014).

13 Zhang, Q. et al. Characterization of the co-elution of host cell proteins with monoclonal antibodies during protein A purification. Biotechnol Prog 32, 708-717, doi:10.1002/btpr.2272 (2016).

14 Seamon, K. B. Specifications for biotechnology-derived protein drugs. Curr Opin Biotechnol 9, 319-325 (1998).

15 Rathore, A. S. Setting Specifications for a Biotech Therapeutic Product in the Quality by Design Paradigm. BioPharm Internaltional 23 (2010). accessible on the world wide web at www dot biopharminternational dot com/setting-specifications-biotech-therapeutic-product-quality-design-paradigm?id=&pageID=1&sk=&date=.

16 Rathore, A. S. Roadmap for implementation of quality by design (QbD) for biotechnology products. Trends Biotechnol 27, 546-553, doi:10.1016/j.tibtech.2009.06.006 (2009).

17 Casadevall, N. et al. Pure red-cell aplasia and antierythropoietin antibodies in patients treated with recombinant erythropoietin. N Engl J Med 346, 469-475, doi:10.1056/NEJMoa011931 (2002).

18 Li, J. et al. Thrombocytopenia caused by the development of antibodies to thrombopoietin. Blood 98, 3241-3248 (2001).

19 Bautista, A. C., Salimi-Moosavi, H. & Jawa, V. Universal immunoassay applied during early development of large molecules to understand impact of immunogenicity on biotherapeutic exposure. AAPS J 14, 843-849, doi:10.1208/s12248-012-9403-0 (2012).

20 Sauerborn, M., Brinks, V., Jiskoot, W. & Schellekens, H. Immunological mechanism underlying the immune response to recombinant human protein therapeutics. Trends Pharmacol Sci 31, 53-59, doi:10.1016/j.tips.2009.11.001 (2010).

21 Baker, M. P., Reynolds, H. M., Lumicisi, B. & Bryson, C. J. Immunogenicity of protein therapeutics: The key causes, consequences and challenges. Self Nonself 1, 314-322, doi:10.4161/self.1.4.13904 (2010).

22 Sauna, Z. E., Lagassé, D., Pedras-Vasconcelos, J., Golding, B. & Rosenberg, A. S. Evaluating and Mitigating the Immunogenicity of Therapeutic Proteins. Trends Biotechnol 36, 1068-1084, doi:10.1016/j.tibtech.2018.05.008 (2018).

23 Mantel, N. Evaluation of survival data and two new rank order statistics arising in its consideration. Cancer Chemother Rep 50, 163-170 (1966).

24 Peto, R. & Peto, J. Asymptotically Efficient Rank Invariant Test Procedures. Journal of the Royal Statistical Society. Series A (General) 135, 185-207, doi:10.2307/2344317 (1972).

25 Sauerborn, M., Brinks, V., Jiskoot, W. & Schellekens, H. Immunological mechanism underlying the immune response to recombinant human protein therapeutics. Trends Pharmacol Sci 31, 53-59, doi:10.1016/j.tips.2009.11.001 (2010).

26 Baker, M. P., Reynolds, H. M., Lumicisi, B. & Bryson, C. J. Immunogenicity of protein therapeutics. The key causes consequences and challenges. Self Nonself 1, 314-322, doi:10.4161/self.1.4.13904 (2010).

27. Wurth C., Demeule B., Mahler H.-C. & Adler M. Quality by Design Approaches to Formulation Robustness - An Antibody Case Study J. Pharma. Sci. 105(5), 1667-1675 (2016). J Z

The use of the terms "a" and "an" and "the" and similar referents in the context of describing the disclosure (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted.

The terms "patient" and "subject" are used interchangeably herein. Generally, these terms will be understood to refer to humans. In the methods of some embodiments, the patient or subject is a human.

Recitation of ranges of values herein are merely intended to serve as a shorthand method of referring individually to each separate value falling within the range and each endpoint, unless otherwise indicated herein, and each separate value and endpoint is incorporated into the specification as if it were individually recited herein.

All methods described herein can be performed in any suitable order unless otherwise indicated herein or otherwise clearly contradicted by context. The use of any and all examples, or exemplary language (e.g., "such as") provided herein, is intended merely to better illuminate the disclosure and does not pose a limitation on the scope of the disclosure unless otherwise claimed. No language in the specification should be construed as indicating any non-claimed element as essential to the practice of the disclosure.

Preferred embodiments of this disclosure are described herein, including the best mode known to the inventors for carrying out claimed invention.

## Claims

1.   A method of manufacturing a biological therapy, the method comprising:

detecting a level of a molecular attribute of the biological therapy in a formulation at one or more timepoints under storage conditions;

determining a rate of change of the molecular attribute under the storage conditions, wherein a molecular attribute is a chemically or physically changed structure of the molecule that defines its physicochemical properties;

obtaining data on *in vivo* safety and/or efficacy of the biological therapy for subjects that have received an administration of the biological therapy;

estimating a level of molecular attribute exposure received by the subjects at the time of said administration, based on (i) the rate of change of the molecular attribute during storage of the biological therapy in the formulation and (ii) a duration that the biological therapy in the formulation was under the storage conditions prior to said administration;

determining a presence or absence of a correlation between the estimated level of molecular attribute exposure and the safety and/or efficacy data for the biological therapy; and

if the correlation is absent, manufacturing production lots of the biological therapy comprising the molecular attribute at or below a specified permissible level of the molecular attribute based on the estimated level of molecule attribute exposure.

2.   The method of claim 1, wherein if the correlation is present, the method further comprises setting a specification for levels of the molecular attribute at the time of manufacture that do not exceed a maximum permissible level of the molecular attribute of the biological therapy,

wherein said maximum permissible level of the molecular attribute is based on the highest estimated levels of molecular attribute exposure that are not associated with adverse events and/or inhibition of efficacy of the biological therapy,

said manufacturing further comprising rejecting production lots of the biological therapy comprising levels of the molecular attribute exceeding the maximum permissible levels.

3.   The method of claim 2, wherein the specified maximum permissible level of the molecular attribute is calculated to produce a level of attribute exposure at end-of-shelf that is less than or equal to 90-100% of the highest estimated level of molecular attribute exposure that is not associated with adverse events and/or an inhibition of efficacy in subjects.

4. A method of developing a manufacturing process for a biological therapy, the method comprising:

detecting a level of a molecular attribute of the biological therapy in a formulation at one or more timepoints under storage conditions;
determining a rate of change of the molecular attribute under the storage conditions;
obtaining data on safety and/or efficacy of the biological therapy in subjects that have received an administration of the biological therapy;
estimating a level of molecular attribute exposure received by the subjects at the time of said administration, based on (i) the rate of change of the molecular attribute during storage of the biological therapy in the formulation and (ii) a duration that the biological therapy in the formulation was under the storage conditions prior to said administration;
determining a presence or absence of a correlation between the estimated level of molecular attribute exposure and the safety and/or efficacy data of the biological therapy; and

(a) if the correlation is absent, establishing the manufacturing process to produce levels of the molecular attribute at or below a specified permissible level based on the estimated level of molecular attribute exposure; or
(b) if the correlation is present, establishing the manufacturing process to produce levels of the molecular attribute at or below a specified a maximum permissible level of the molecular attribute based on the highest level of the molecular attribute that is not associated with adverse events and/or inhibition of efficacy of the biological therapy, wherein a molecular attribute is a chemically or physically changed structure of the molecule that defines its physicochemical properties.

5. A method of assessing a clinical impact of a molecular attribute of a biological therapy, the method comprising:

detecting a level of a molecular attribute of the biological therapy in a formulation at one or more timepoints under storage conditions;
determining a rate of change of the molecular attribute under the storage conditions;
obtaining data on safety and/or efficacy of the biological therapy in subjects that have received an administration of the biological therapy;
estimating a level of molecular attribute exposure received by the subjects at the time of said administration, based on (i) the rate of change of the molecular attribute during storage of the biological therapy in the formulation and (ii) a duration that the biological therapy in the formulation was under the storage conditions prior to said administration;
determining a presence or absence of a correlation between the estimated molecular attribute exposure and the safety and/or efficacy of the biological therapy; and
if (a) the correlation is absent, determining that the molecular attribute impacts neither clinical safety nor efficacy of the biological therapy; or
if (b) the correlation is present, determining that the molecular attribute impacts safety and/or efficacy of the biological therapy, wherein a molecular attribute is a chemically or physically changed structure of the molecule that defines its physicochemical properties.

6. The method of claim 5, wherein if (a) the correlation is absent, the method further comprises setting a specification for permissible levels of the molecular attribute of the biological therapy, wherein the permissible levels of the molecular attribute are based on the highest estimated levels of molecular attribute exposure received by the subjects, or wherein (b) if the correlation is present, the method further comprises setting a specification for maximum permissible levels of the molecular attribute of the biological therapy, wherein said maximum permissible levels of the molecular attribute are based on levels of the molecular attribute associated with adverse events and/or inhibition of efficacy of the biological therapy.

7. The method of any one of the preceding claims, wherein

a) the estimating is further based on (iii) a dose of the biological therapy in said administration, and (iv) an amount of the molecular attribute measured at time of manufacture and/or lot release;
b) estimating the level of the molecular attribute exposure comprises the calculation:

$$A_t = \left\{ \%A_0 + \sum_{i=1}^{n} \left( \%A_{\Delta_i} \times t_i \right) \right\} \times D$$

wherein $A_t$ is the estimated level of molecular attribute exposure, $\%A_0$ is the percentage of the molecular attribute at time of lot release, $\%A_{\Delta i}$ is the rate of change of the percentage of the molecular attribute level over time at a given storage condition, $t_i$ is the time of storage at the given condition, and D is a dose strength of the administration; and/or

c) estimating the level of the molecular attribute exposure comprises the calculation:

$$\%A_{\text{rel.}} = \frac{A_t}{D} \times 100\%$$

wherein $\%A_{\text{rel.}}$ is percentage of relative level of the molecular attribute exposure with respect to dosage, wherein $A_t$ is level of the molecular attribute exposure computed using the equation 1 or 2, and wherein D is dose strength in the dimension of weigh of active pharmaceutical ingredient associated with each treatment.

8. The method of any one of the preceding claims, wherein

a) the level of the molecular attribute of the biological therapy in the formulation is detected at two or more timepoints under storage conditions; and/or
b) the molecular attribute comprises at least one of: acidic species, basic species, high molecular weight species, subvisible particle number, low molecular weight, middle molecular weight, glycosylation (such as non-glycosylated heavy chain or high mannose), non-heavy chain and light chain, deamidation, deamination, cyclization, oxidation, isomerization, fragmentation/clipping, N-terminal and C-terminal variants, reduced and partial species, folded structure, surface hydrophobicity, chemical modification, covalent bond, a C-terminal amino acid motif PARG, or a C-terminal amino acid motif PAR-Amide.

9. The method of any one of the preceding claims, wherein the one or more timepoints or two or more timepoints comprise a time of manufacture, and at least two subsequent timepoints.

10. The method of any one of the preceding claims, wherein

a) the correlation comprises a weighted correlation between attribute exposure and adverse event occurrences; and/or
b) determining a presence or absence of a correlation between the estimated level of molecular attribute exposure and the safety and/or efficacy data for the biological therapy comprises Bayesian estimation.

11. The method of any one of the preceding claims, wherein

a) the biological therapy in the formulation was under the storage conditions for different durations in the administrations to different subjects;
b) the administration comprises two or more administration events, preferably wherein the estimated level of molecular attribute exposure received by the subjects at the time of said administration is a maximum or an average of the two or more administration events; and/or
c) said administration comprises a continuous infusion, and said estimating comprises calculating an estimated level of molecular attribute exposure during two or more intervals of said continuous infusion, such 24-hour intervals.

12. The method of any one of the preceding claims, wherein

a) said safety data comprise adverse event data, preferably wherein the safety data comprises an adverse event time course; and/or
b) said efficacy data comprise clinical endpoint data.

13. The method of any one of the preceding claims, wherein the biological therapy is selected from the group consisting of: an antibody, an antigen-binding antibody fragment, an antibody protein product, a Bi-specific T cell engager (BiTE®)

molecule, a bispecific antibody, a trispecific antibody, an Fc fusion protein, a recombinant protein, a recombinant virus, a recombinant T cell, a synthetic peptide, and an active fragment of a recombinant protein.

14. The method of any one of the preceding claims, wherein the formulation is a pharmaceutically acceptable formulation.

15. The method of any one of the preceding claims, wherein detecting the level of a molecular attribute of the biological therapy comprises mass spectrometry, chromatography, electrophoresis, spectroscopy, light obscuration, a particle method (such as nanoparticle/visible/micron-sized resonant mass or Brownian motion), analytical centrifugation, imaging or imaging characterization, or immunoassay.

**Patentansprüche**

1. Ein Verfahren zur Herstellung einer biologischen Therapie, wobei das Verfahren umfasst:

Nachweis des Gehalts eines molekularen Attributs der biologischen Therapie in einer Formulierung zu einem oder mehreren Zeitpunkten unter Lagerungsbedingungen;
Bestimmen einer Änderungsrate des molekularen Attributs unter den Lagerungsbedingungen, wobei ein molekulares Attribut eine chemisch oder physikalisch veränderte Struktur des Moleküls ist, die seine physiochemischen Eigenschaften definiert;
Gewinnung von Daten zur In-vivo-Sicherheit und/oder -Wirksamkeit der biologischen Therapie bei Probanden, die eine Verabreichung der biologischen Therapie erhalten haben;
Abschätzung des Ausmaßes der Exposition gegenüber dem molekularen Attribut, das die Versuchspersonen zum Zeitpunkt der Verabreichung erhalten haben, basierend auf (i) der Änderungsrate des molekularen Attributs während der Lagerung der biologischen Therapie in der Formulierung und (ii) einer Dauer, die die biologische Therapie in der Formulierung unter den Lagerungsbedingungen vor der Verabreichung war;
Bestimmung des Vorhandenseins oder Nichtvorhandenseins einer Korrelation zwischen dem geschätzten Niveau der molekularen Attributexposition und den Sicherheits- und/oder Wirksamkeitsdaten für die biologische Therapie; und
wenn die Korrelation nicht vorhanden ist, die Herstellung von Produktionschargen der biologischen Therapie, die das molekulare Attribut enthält, bei oder unterhalb eines spezifizierten zulässigen Niveaus des molekularen Attributs auf der Grundlage des geschätzten Niveaus der Molekülattribut-Exposition.

2. Das Verfahren nach Anspruch 1, wobei, wenn die Korrelation vorhanden ist, das Verfahren ferner das Festlegen einer Spezifikation für die Gehalte des molekularen Attributs zum Zeitpunkt der Herstellung umfasst, die einen maximal zulässigen Gehalt des molekularen Attributs der biologischen Therapie nicht überschreitet,

wobei das maximal zulässige Niveau des molekularen Attributs auf den höchsten geschätzten Niveaus der molekularen Attributexposition basiert, die nicht mit unerwünschten Ereignissen und/oder einer Hemmung der Wirksamkeit der biologischen Therapie verbunden sind,
wobei die Herstellung ferner das Zurückweisen von Produktionschargen der biologischen Therapie umfasst, die Konzentrationen des molekularen Attributs aufweisen, die die maximal zulässigen Konzentrationen überschreiten.

3. Das Verfahren nach Anspruch 2, wobei der spezifizierte maximal zulässige Gehalt des molekularen Attributs so berechnet wird, dass ein Gehalt an Attribut-Exposition am Ende der Haltbarkeit erreicht wird, der weniger als oder gleich 90-100% des höchsten geschätzten Gehalts an molekularer Attribut-Exposition beträgt, der nicht mit unerwünschten Ereignissen und/oder einer Hemmung der Wirksamkeit bei Probanden verbunden ist.

4. Ein Verfahren zur Entwicklung eines Herstellungsprozesses für eine biologische Therapie, wobei das Verfahren umfasst:

Nachweis des Gehalts eines molekularen Attributs der biologischen Therapie in einer Formulierung zu einem oder mehreren Zeitpunkten unter Lagerungsbedingungen;
Bestimmung der Änderungsrate des molekularen Attributs unter den Lagerungsbedingungen;
Gewinnung von Daten zur Sicherheit und/oder Wirksamkeit der biologischen Therapie bei Probanden, die eine Verabreichung der biologischen Therapie erhalten haben;
Abschätzung des Ausmaßes der Exposition gegenüber dem molekularen Attribut, das die Versuchspersonen zum Zeitpunkt der Verabreichung erhalten haben, basierend auf (i) der Änderungsrate des molekularen Attributs während der Lagerung der biologischen Therapie in der Formulierung und (ii) einer Dauer, die die biologische Therapie in der Formulierung unter den Lagerungsbedingungen vor der Verabreichung war;

Bestimmung des Vorhandenseins oder Nichtvorhandenseins einer Korrelation zwischen dem geschätzten Niveau der molekularen Attributbelastung und den Sicherheits- und/oder Wirksamkeitsdaten der biologischen Therapie; und

(a) bei Fehlen der Korrelation: Festlegung des Herstellungsverfahrens zur Erzeugung von Gehalten des molekularen Attributs, die auf der Grundlage des geschätzten Gehalts der Exposition gegenüber dem molekularen Attribut auf oder unter einem bestimmten zulässigen Niveau liegen; oder

(b) wenn die Korrelation vorhanden ist, Einrichten des Herstellungsverfahrens zur Herstellung von Konzentrationen des molekularen Attributs bei oder unter einer spezifizierten maximal zulässigen Konzentration des molekularen Attributs, basierend auf der höchsten Konzentration des molekularen Attributs, die nicht mit unerwünschten Ereignissen und/oder einer Hemmung der Wirksamkeit der biologischen Therapie verbunden ist, wobei ein molekulares Attribut eine chemisch oder physikalisch veränderte Struktur des Moleküls ist, die seine physiochemischen Eigenschaften definiert.

5. Ein Verfahren zur Bewertung der klinischen Auswirkungen eines molekularen Attributs einer biologischen Therapie, wobei das Verfahren umfasst:

Nachweis des Gehalts eines molekularen Attributs der biologischen Therapie in einer Formulierung zu einem oder mehreren Zeitpunkten unter Lagerungsbedingungen;

Bestimmung der Änderungsrate des molekularen Attributs unter den Lagerungsbedingungen;

Gewinnung von Daten zur Sicherheit und/oder Wirksamkeit der biologischen Therapie bei Probanden, die eine Verabreichung der biologischen Therapie erhalten haben;

Abschätzung des Ausmaßes der Exposition des molekularen Attributs, das die Probanden zum Zeitpunkt der Verabreichung erhalten haben, basierend auf (i) der Änderungsrate des molekularen Attributs während der Lagerung der biologischen Therapie in der Formulierung und (ii) einer Dauer, die die biologische Therapie in der Formulierung unter den Lagerungsbedingungen vor der Verabreichung war;

Bestimmung des Vorhandenseins oder Fehlens einer Korrelation zwischen der geschätzten molekularen Attributbelastung und der Sicherheit und/oder Wirksamkeit der biologischen Therapie; und

wenn a) die Korrelation nicht vorhanden ist, die Feststellung, dass das molekulare Merkmal weder die klinische Sicherheit noch die Wirksamkeit der biologischen Therapie beeinflusst; oder

wenn (b) die Korrelation vorhanden ist, Bestimmung, dass das molekulare Attribut die Sicherheit und/oder Wirksamkeit der biologischen Therapie beeinflusst, wobei ein molekulares Attribut eine chemisch oder physikalisch veränderte Struktur des Moleküls ist, die seine physiochemischen Eigenschaften definiert.

6. Das Verfahren nach Anspruch 5, wobei, wenn (a) die Korrelation nicht vorhanden ist, das Verfahren ferner das Festlegen einer Spezifikation für zulässige Konzentrationen des molekularen Attributs der biologischen Therapie umfasst, wobei die zulässigen Konzentrationen des molekularen Attributs auf den höchsten geschätzten Konzentrationen der Exposition des molekularen Attributs basieren, die die Probanden erhalten, oder

wobei (b) wenn die Korrelation vorhanden ist, das Verfahren ferner das Festlegen einer Spezifikation für maximal zulässige Spiegel des molekularen Attributs der biologischen Therapie umfasst, wobei die maximal zulässigen Spiegel des molekularen Attributs auf Spiegeln des molekularen Attributs beruhen, die mit unerwünschten Ereignissen und/oder einer Hemmung der Wirksamkeit der biologischen Therapie verbunden sind.

7. Das Verfahren nach einem der vorhergehenden Ansprüche, wobei

a) Die Schätzung basiert ferner auf (iii) einer Dosis der biologischen Therapie bei der Verabreichung und (iv) einer Menge des molekularen Attributs, die zum Zeitpunkt der Herstellung und/oder der Chargenfreigabe gemessen wurde;

b) die Abschätzung der Höhe der Exposition des molekularen Attributs die folgende Berechnung umfasst:

$$A_t = \left\{ \%A_0 + \sum_{i=1}^{n} \left( \%A_{\Delta_i} \times t_i \right) \right\} \times D$$

wobei $A_t$ die geschätzte Höhe der Exposition gegenüber dem molekularen Attribut ist, $\%A_0$ der Prozentsatz des

molekularen Attributs zum Zeitpunkt der Chargenfreigabe ist, %A$_{\Delta i}$ die Änderungsrate des Prozentsatzes des molekularen Attributniveaus über die Zeit bei einer gegebenen Lagerungsbedingung ist, t$_i$ die Lagerungszeit bei der gegebenen Bedingung ist und D eine Dosisstärke der Verabreichung ist; und/oder

c) die Abschätzung der Höhe der Exposition des molekularen Attributs die folgende Berechnung umfasst:

$$\%A_{\text{rel.}} = \frac{A_t}{D} \times 100\%,$$

wobei *%A*$_{\text{rel.}}$ der Prozentsatz des relativen Niveaus der Exposition gegenüber dem molekularen Attribut in Bezug auf die Dosierung ist, wobei $A_t$ das Niveau der Exposition gegenüber dem molekularen Attribut ist, das unter Verwendung der Gleichung 1 oder 2 berechnet wird, und wobei D die Dosisstärke in der Dimension des Gewichts des aktiven pharmazeutischen Inhaltsstoffs ist, der mit jeder Behandlung verbunden ist.

8. Das Verfahren nach einem der vorhergehenden Ansprüche, wobei

a) der Gehalt des molekularen Attributs der biologischen Therapie in der Formulierung zu zwei oder mehr Zeitpunkten unter Lagerbedingungen nachgewiesen wird; und/oder

b) das molekulare Attribut mindestens eines der folgenden Merkmale umfasst: saure Spezies, basische Spezies, Spezies mit hohem Molekulargewicht, subvisible Teilchenzahl, niedriges Molekulargewicht, mittleres Molekulargewicht, Glykosylierung (wie nicht-glykosylierte schwere Kette oder hohe Mannose), nicht-schwere Kette und leichte Kette, Deamidierung, Desaminierung, Zyklisierung, Oxidation, Isomerisierung, Fragmentierung/Clipping, N-terminale und C-terminale Varianten, reduzierte und partielle Spezies, gefaltete Struktur, Oberflächenhydrophobizität, chemische Modifikation, kovalente Bindung, ein C-terminales Aminosäuremotiv PARG oder ein C-terminales Aminosäuremotiv PAR-Amid.

9. Das Verfahren nach einem der vorhergehenden Ansprüche, wobei der eine oder die mehreren Zeitpunkte oder zwei oder mehrere Zeitpunkte einen Herstellungszeitpunkt und mindestens zwei aufeinander folgende Zeitpunkte umfassen.

10. Das Verfahren nach einem der vorhergehenden Ansprüche, wobei

a) die Korrelation eine gewichtete Korrelation zwischen der Exposition gegenüber einem Attribut und dem Auftreten eines unerwünschten Ereignisses umfasst; und/oder

b) die Bestimmung des Vorhandenseins oder Nichtvorhandenseins einer Korrelation zwischen dem geschätzten Grad der Exposition gegenüber einem molekularen Attribut und den Sicherheits- und/oder Wirksamkeitsdaten für die biologische Therapie eine Bayes'sche Schätzung umfasst.

11. Das Verfahren nach einem der vorhergehenden Ansprüche, wobei

a) die biologische Therapie in der Formulierung bei den Verabreichungen an verschiedene Probanden unterschiedlich lange den Lagerungsbedingungen ausgesetzt war;

b) die Verabreichung zwei oder mehr Verabreichungsereignisse umfasst, wobei vorzugsweise das geschätzte Niveau der Exposition gegenüber dem molekularen Attribut, das die Probanden zum Zeitpunkt der Verabreichung erhalten, ein Maximum oder ein Durchschnitt der zwei oder mehr Verabreichungsereignisse ist; und/oder

c) die Verabreichung eine kontinuierliche Infusion umfasst und die Schätzung die Berechnung eines geschätzten Niveaus der Exposition gegenüber dem molekularen Attribut während zwei oder mehr Intervallen der kontinuierlichen Infusion, wie beispielsweise 24-Stunden-Intervallen, umfasst.

12. Das Verfahren nach einem der vorhergehenden Ansprüche, wobei

a) die Sicherheitsdaten Daten über unerwünschte Ereignisse umfassen, wobei die Sicherheitsdaten vorzugsweise einen Zeitverlauf der unerwünschten Ereignisse umfassen; und/oder

b) die Wirksamkeitsdaten klinische Endpunktdaten umfassen.

13. Das Verfahren nach einem der vorhergehenden Ansprüche, wobei die biologische Therapie ausgewählt ist aus der Gruppe bestehend aus: einem Antikörper, einem Antigenbindenden Antikörperfragment, einem Antikörperproteinprodukt, einem Bi-spezifischen T-Zell-Engager (BiTEO)-Molekül, einem bispezifischen Antikörper, einem trispezifischen Antikörper, einem Fc-Fusionsprotein, einem rekombinanten Protein, einem rekombinanten Virus, einer rekombinanten T-Zelle, einem synthetischen Peptid und einem aktiven Fragment eines rekombinanten Proteins.

14. Das Verfahren nach einem der vorhergehenden Ansprüche, wobei die Formulierung eine pharmazeutisch akzeptable Formulierung ist.

15. Das Verfahren nach einem der vorhergehenden Ansprüche, wobei der Nachweis des Gehalts eines molekularen Attributs der biologischen Therapie Massenspektrometrie, Chromatographie, Elektrophorese, Spektroskopie, Lichtverschleierung, eine Partikelmethode (wie Nanopartikel/sichtbare/mikroskopische Resonanzmasse oder Brownsche Bewegung), analytische Zentrifugation, Bildgebung oder Bildcharakterisierung oder Immunoassay umfasst.

**Revendications**

1. Procédé de fabrication d'une thérapie biologique, le procédé comprenant les étapes consistant à :

   détecter un niveau d'un attribut moléculaire de la thérapie biologique dans une formulation au niveau d'un ou de plusieurs points dans le temps sous des conditions de stockage ;
   déterminer un taux de changement de l'attribut moléculaire sous les conditions de stockage, dans lequel un attribut moléculaire est une structure de la molécule chimiquement ou physiquement modifiée qui définit ses propriétés physicochimiques ;
   obtenir des données sur une sécurité et/ou une efficacité *in vivo* de la thérapie biologique pour des sujets qui ont reçu une administration de la thérapie biologique ;
   estimer un niveau d'exposition à l'attribut moléculaire reçue par les sujets au moment de ladite administration, sur la base (i) du taux de changement de l'attribut moléculaire durant un stockage de la thérapie biologique dans la formulation et (ii) une durée durant laquelle la thérapie biologique dans la formulation se trouvait sous les conditions de stockage avant ladite administration ;
   déterminer une présence ou une absence d'une corrélation entre le niveau estimé de l'exposition à l'attribut moléculaire et les données de sécurité et/ou d'efficacité pour la thérapie biologique ; et
   si la corrélation est absente, fabriquer des lots de production de la thérapie biologique comprenant l'attribut moléculaire au niveau, ou en-dessous, d'un niveau permissible spécifié de l'attribut moléculaire sur la base du niveau estimé de l'exposition à l'attribut moléculaire.

2. Procédé selon la revendication 1, dans lequel si la corrélation est présente, le procédé comprend en outre la définition d'une spécification pour des niveaux de l'attribut moléculaire au moment de la fabrication qui n'excèdent pas un niveau permissible maximal de l'attribut moléculaire de la thérapie biologique,

   dans lequel ledit niveau permissible maximal de l'attribut moléculaire est basé sur les niveaux estimés les plus élevés de l'exposition à l'attribut moléculaire qui ne sont pas associés à des événements adverses et/ou à une inhibition de l'efficacité de la thérapie biologique,
   ladite fabrication comprenant en outre le rejet de lots de production de la thérapie biologique comprenant des niveaux de l'attribut moléculaire excédant les niveaux permissibles maximaux.

3. Procédé selon la revendication 2, dans lequel le niveau permissible maximal spécifié de l'attribut moléculaire est calculé pour produire un niveau d'exposition à l'attribut à la péremption du produit qui est inférieur ou égal à 90 à 100 % du niveau estimé le plus élevé de l'exposition à l'attribut moléculaire qui n'est pas associé à des événements adverses et/ou à une inhibition de l'efficacité chez des sujets.

4. Procédé de développement d'un procédé de fabrication pour une thérapie biologique, le procédé comprenant les étapes consistant à :

   détecter un niveau d'un attribut moléculaire de la thérapie biologique dans une formulation au niveau d'un ou de plusieurs points dans le temps sous des conditions de stockage ;
   déterminer un taux de changement de l'attribut moléculaire sous les conditions de stockage ;
   obtenir des données sur la sécurité et/ou l'efficacité de la thérapie biologique chez les sujets qui ont reçu une administration de la thérapie biologique ;
   estimer un niveau d'exposition à l'attribut moléculaire reçue par les sujets au moment de ladite administration, sur la base (i) du taux de changement de l'attribut moléculaire durant un stockage de la thérapie biologique dans la formulation et (ii) une durée durant laquelle la thérapie biologique dans la formulation se trouvait sous les conditions de stockage avant ladite administration ;

déterminer une présence ou une absence d'une corrélation entre le niveau estimé de l'exposition à l'attribut moléculaire et les données de sécurité et/ou d'efficacité de la thérapie biologique ; et

(a) si la corrélation est absente, établir le procédé de fabrication pour produire des niveaux de l'attribut moléculaire au niveau, ou en-dessous, d'un niveau permissible spécifié sur la base du niveau estimé de l'exposition à l'attribut moléculaire ; ou

(b) si la corrélation est présente, établir le procédé de fabrication pour produire des niveaux de l'attribut moléculaire au niveau, ou en-dessous, d'un niveau permissible maximal spécifié de l'attribut moléculaire sur la base du niveau le plus élevé de l'attribut moléculaire qui n'est pas associé à des événements adverses et/ou une inhibition de l'efficacité de la thérapie biologique, dans lequel un attribut moléculaire est une structure de la molécule chimiquement ou physiquement modifiée qui définit ses propriétés physicochimiques.

5. Procédé d'évaluation d'un impact clinique d'un attribut moléculaire d'une thérapie biologique, le procédé comprenant les étapes consistant à :

détecter un niveau d'un attribut moléculaire de la thérapie biologique dans une formulation au niveau d'un ou de plusieurs points dans le temps sous des conditions de stockage ;

déterminer un taux de changement de l'attribut moléculaire sous les conditions de stockage ;

obtenir des données sur la sécurité et/ou l'efficacité de la thérapie biologique chez des sujets qui ont reçu une administration de la thérapie biologique ;

estimer un niveau d'exposition à l'attribut moléculaire reçue par les sujets au moment de ladite administration, sur la base (i) du taux de changement de l'attribut moléculaire durant un stockage de la thérapie biologique dans la formulation et (ii) une durée durant laquelle la thérapie biologique dans la formulation se trouvait sous les conditions de stockage avant ladite administration ;

déterminer une présence ou une absence d'une corrélation entre l'exposition estimée à l'attribut moléculaire et la sécurité et/ou l'efficacité de la thérapie biologique ; et

si (a) la corrélation est absente, déterminer que l'attribut moléculaire n'impacte ni la sécurité ni l'efficacité clinique de la thérapie biologique ; ou

si (b) la corrélation est présente, déterminer que l'attribut moléculaire impacte une sécurité et/ou une efficacité de la thérapie biologique, dans lequel un attribut moléculaire est une structure de la molécule chimiquement ou physiquement modifiée qui définit ses propriétés physicochimiques.

6. Procédé selon la revendication 5, dans lequel si (a) la corrélation est absente, le procédé comprend en outre la définition d'une spécification pour des niveaux permissibles de l'attribut moléculaire de la thérapie biologique, dans lequel les niveaux permissibles de l'attribut moléculaire sont basés sur les niveaux estimés les plus élevés de l'exposition à l'attribut moléculaire reçue par les sujets, ou

dans lequel (b) si la corrélation est présente, le procédé comprend en outre la définition d'une spécification pour des niveaux permissibles maximaux de l'attribut moléculaire de la thérapie biologique, dans lequel lesdits niveaux permissibles maximaux de l'attribut moléculaire sont basés sur des niveaux de l'attribut moléculaire associés à des événements adverses et/ou à une inhibition de l'efficacité de la thérapie biologique.

7. Procédé selon l'une quelconque des revendications précédentes, dans lequel

a) l'estimation est en outre basée sur (iii) une dose de la thérapie biologique dans ladite administration, et (iv) une quantité de l'attribut moléculaire mesurée au moment de la fabrication et/ou de la mise sur le marché du lot ;

b) l'estimation du niveau de l'exposition à l'attribut moléculaire comprend le calcul :

$$A_t = \{\%A_0 + \sum_{i=1}^{n} (\%A_{\Delta i} \ \text{x} \ t_i)\} \ \text{x} \ D$$

dans lequel $A_t$ est le niveau estimé de l'exposition à l'attribut moléculaire, $\%A_0$ est le pourcentage de l'attribut moléculaire au moment de la mise sur le marché du lot, $\%A_{\Delta i}$ est le taux de changement du pourcentage du niveau de l'attribut moléculaire dans le temps sous une condition donnée de stockage, $t_i$ est la durée de stockage à la condition donnée, et $D$ est une puissance de dose de l'administration ; et/ou

c) l'estimation du niveau de l'exposition à l'attribut moléculaire comprend le calcul :

$$\%A_{\text{rel.}} = \frac{A_t}{D} \text{ x } 100\%$$

dans lequel $\%A_{\text{rel.}}$ est un pourcentage du niveau relatif de l'exposition à l'attribut moléculaire par rapport à la dose, dans lequel $A_t$ est le niveau de l'exposition à l'attribut moléculaire calculé en utilisant l'équation 1 ou 2, et dans lequel D est la puissance du dosage dans la dimension de poids de l'ingrédient pharmaceutique actif associé à chaque traitement.

8. Procédé selon l'une quelconque des revendications précédentes, dans lequel

a) le niveau de l'attribut moléculaire de la thérapie biologique dans la formulation est détecté à deux ou plusieurs points dans le temps sous des conditions de stockage ; et/ou
b) l'attribut moléculaire comprend au moins l'un de : des espèces acides, des espèces basiques, des espèces à poids moléculaire élevé, un nombre de particules pouvant être subdivisé, un faible poids moléculaire, un poids moléculaire modéré, une glycosylation (telle qu'un mannose non glycosylé à chaîne lourde ou élevée), une chaîne non lourde et une chaîne légère, une désamidation, déamination, cyclisation, oxydation, isomérisation, fragmentation/découpage, des variants en terminaison N et en terminaison C, des espèces réduites et partielles, une structure pliée, une hydrophobie de surface, une modification chimique, une liaison covalente, un motif PARG d'acide aminé en terminaison C, ou un motif PAR-amide d'acide aminé en terminaison C.

9. Procédé selon l'une quelconque des revendications précédentes, dans lequel le un ou plusieurs points dans le temps ou les deux ou plusieurs points dans le temps comprennent un moment de fabrication, et au moins deux points dans le temps ultérieurs.

10. Procédé selon l'une quelconque des revendications précédentes, dans lequel

a) la corrélation comprend une corrélation pondérée entre une exposition à l'attribut et des occurrences d'évènement adverse ; et/ou
b) une détermination d'une présence ou d'une absence d'une corrélation entre le niveau estimé de l'exposition à l'attribut moléculaire et les données de sécurité et/ou d'efficacité pour la thérapie biologique comprend une estimation bayésienne.

11. Procédé selon l'une quelconque des revendications précédentes, dans lequel

a) la thérapie biologique dans la formulation se trouvait sous des conditions de stockage pendant des durées différentes dans les administrations à différents sujets ;
b) l'administration comprend deux ou plusieurs évènements d'administration, préférablement dans lequel le niveau estimé de l'exposition à l'attribut moléculaire reçue par les sujets au moment de ladite administration est un maximum ou une moyenne de deux ou plusieurs évènements d'administration ; et/ou
c) ladite administration comprend une perfusion continue, et ladite estimation comprend un calcul d'un niveau estimé d'exposition à l'attribut moléculaire durant deux ou plusieurs intervalles de ladite perfusion continue, tels que des intervalles de 24 heures.

12. Procédé selon l'une quelconque des revendications précédentes, dans lequel

a) lesdites données de sécurité comprennent des données d'évènement adverse, préférablement dans lequel les données de sécurité comprennent un cours du temps d'évènement adverse ; et/ou
b) lesdites données d'efficacité comprennent des données de point final clinique.

13. Procédé selon l'une quelconque des revendications précédentes, dans lequel la thérapie biologique est sélectionnée dans le groupe constitué : d'un anticorps, d'un fragment d'anticorps de liaison à l'antigène, d'un produit protéique d'anticorps, d'une molécule d'engagement des cellules T bispécifiques (BiTE®), d'un anticorps bispécifique, d'un anticorps trispécifique, d'une protéine de fusion Fc, d'une protéine recombinante, d'un virus recombinant, d'une cellule T recombinante, d'un peptide synthétique, et d'un fragment actif d'une protéine recombinante.

14. Procédé selon l'une quelconque des revendications précédentes, dans lequel la formulation est une formulation pharmaceutiquement acceptable.

15. Procédé selon l'une quelconque des revendications précédentes, dans lequel une détection du niveau d'un attribut moléculaire de la thérapie biologique comprend une spectrométrie de masse, chromatographie, électrophorèse, spectroscopie, un obscurcissement de la lumière, un procédé de particules (tel qu'une masse résonante de nanoparticule/visible/de la taille d'un micron ou un mouvement brownien), une centrifugation analytique, une imagerie ou caractérisation par imagerie, ou un dosage immunologique.

# FIG. 1A

% Level of Attribute at Time of Manufacture ($\%A_0$)

% Level of Attribute at Time of Administration ($\%A_t$)

Level of Attribute

% Change in Attribute Level ($\%A_\Delta$)

Time of Manufacture

Time of Administration

# FIG. 1B

Representative $A_t$ for Individual Patients

ADA-Positive Patients

ADA-Negative Patients

FIG. 1C

Patients with High $A_t$

Patients with Mid-High $A_t$

Patients with Low-Mid $A_t$

Patients with Low $A_t$

%ADA-Positive Patient

Elapsed Time in Clinical Study

Marker Size: Group Size

Group Average of Representive $A_t$

%ADA-Positive Patient

# FIG. 2A

# FIG. 2B

EP 4 262 859 B1

FIG. 3A

# FIG. 3B

EP 4 262 859 B1

# FIG. 4A

SEC HMW

0.82 ± 0.02(256)

1.1 ± 0.1(165)

1.62 ± 0.08(103)

1.37 ± 0.07(103)

rCE-SDS NonHC+LC

1.3 ± 0.1(152)

1.03 ± 0.03(240)

2.1 ± 0.1(111)

1.7 ± 0.1(124)

%ADA-Positive Patient

CEX Acidic Species

22.3 ± 0.1(256)

27 ± 2(117)

42 ± 3(154)

34 ± 2(100)

HIC Pre-Peak

2.94 ± 0.04(6)

3.10 ± 0.05(7)

3.34 ± 0.07(6)

3.6 ± 0.1(5)

EP 4 262 859 B1

# FIG. 4B

Elapsed Time in Clinical Study (month)

EP 4 262 859 B1

FIG. 5A

# FIG. 5B

# FIG. 5C

EP 4 262 859 B1

# FIG. 6

o ADA-Negative Patients
□ ADA-Positive Patients

# FIG. 7A

SEC HMW

rCE-SDS NonHC+LC

CEX Acidic Species

HIC Pre-Peak

o ADA-Negative Patients
□ ADA-Positive Patients

# FIG. 7B

# FIG. 8

SEC HMW

# FIG. 9

EP 4 262 859 B1

# FIG. 10A

EP 4 262 859 B1

# FIG. 10B

Anemia · Cytokine Release Syndrome · Fever · Infusion Related Reaction · Lymphocytopenia · Neurological Event

Scaled Dose per Potency Value (µg/day) · Treatment Dose (µg/day)

Patients with No or Gr ≤ 2 Adverse Events
**Patients with Gr≥3 Adverse Events**

EP 4 262 859 B1

# FIG. 11A

Conventional CIA

Existing CIA Data Set Showing
No Correlation between HMW and Pyrexia

no positive correlation
(p=0.056)

Max. Daily HMW Exposure (µg)

Study Subject Grp per
Pyrexia Adverse Event Occurrence

# FIG. 11C

CIA via Bayesian Approach

Probability Density Function

p-value: 0.374

HMW -Pyrexia Association
Potential

# FIG. 11B

Arbitrary CIA Data Set Modeled to show
Correlation between HMW and Pyrexia

positive correlation with
p=0.050

Max. Daily HMW Exposure (µg)

Study Subject Grp per
Pyrexia Adverse Event Occurrence

# FIG. 11D

Probability Density Function

p-value: $1.06 \times 10^{-8}$

HMW -Pyrexia Association
Potential

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63126274
- US 63242395

### Non-patent literature cited in the description

- **SHIMAMOTO et al.** mAbs, 2012, vol. 4 (5), 586-591
- REMINGTON'S PHARMACEUTICAL SCIENCES. Mack Publishing Company, 1990
- **ROSENBERG, A. S.** ; **VERTHELYI, D.** ; **CHERNEY, B. W.** Managing uncertainty: a perspective on risk pertaining to product quality attributes as they bear on immunogenicity of therapeutic proteins. J Pharm Sci, 2012, vol. 101, 3560-3567
- **GOETZE, A. M.** ; **SCHENAUER, M. R.** ; **FLYNN, G. C.** Assessing monoclonal antibody product quality attribute criticality through clinical studies. MAbs, 2010, vol. 2, 500-507
- **KELLEY, B** ; **CROMWELL, M.** ; **JERKINS, J.** Integration of QbD risk assessment tools and overall risk management.. Biologicals, 2016, vol. 44, 341-351
- **BESSA, J. et al.** The immunogenicity of antibody aggregates in a novel transgenic mouse model.. Pharm Res, 2015, vol. 32, 2344-2359
- **BI, V. et al.** Development of a human antibody tolerant mouse model to assess the immunogenicity risk due to aggregated biotherapeutics.. J Pharm Sci, 2013, vol. 102, 3545-3555
- **BEE, J. S.** ; **GOLETZ, T. J.** ; **RAGHEB, J. A.** The future of protein particle characterization and understanding its potential to diminish the immunogenicity of biopharmaceuticals: a shared perspective.. J Pharm Sci, 2012, vol. 101, 3580-3585
- **GOETZE, A. M.** ; **LIU, Y. D** ; **ARROLL, T.** ; **CHU, L.** ; **FLYNN, G. C.** Rates and impact of human antibody glycation in vivo. Glycobiology, 2012, vol. 22, 221-234
- **JAWA, V. et al.** Evaluating Immunogenicity Risk Due to Host Cell Protein Impurities in Antibody-Based Biotherapeutics.. AAPS J, 2016, vol. 18, 1439-1452
- **JOUBERT, M. K. et al.** Use of In Vitro Assays to Assess Immunogenicity Risk of Antibody-Based Biotherapeutics.. PLoS One, 2016, vol. 11, e0159328
- **LIU, Y. D. et al.** Human IgG2 antibody disulfide rearrangement in vivo.. J Biol Chem, 2008, vol. 283, 29266-29272
- **LIU, Y. D.** ; **VAN ENK, J. Z.** ; **FLYNN, G. C.** Human antibody Fc deamidation in vivo. Biologicals, 2009, vol. 37, 313-322
- **YANG, J.** ; **GOETZE, A. M.** ; **FLYNN, G. C.** Assessment of naturally occurring covalent and total dimer levels in human IgG1 and IgG2.. Mol Immunol, 2014, vol. 58, 108-115
- **ZHANG, Q. et al.** Characterization of the co-elution of host cell proteins with monoclonal antibodies during protein A purification.. Biotechnol Prog, 2016, vol. 32, 708-717
- **SEAMON, K. B.** Specifications for biotechnology-derived protein drugs.. Curr Opin Biotechnol, 1998, vol. 9, 319-325
- **RATHORE, A. S.** Setting Specifications for a Biotech Therapeutic Product in the Quality by Design Paradigm.. BioPharm Internaltional, 2010, vol. 23
- **RATHORE, A. S.** Roadmap for implementation of quality by design (QbD) for biotechnology products.. Trends Biotechnol, 2009, vol. 27, 546-553
- **CASADEVALL, N. et al.** Pure red-cell aplasia and antierythropoietin antibodies in patients treated with recombinant erythropoietin.. N Engl J Med, 2002, vol. 346, 469-475
- **LI, J. et al.** Thrombocytopenia caused by the development of antibodies to thrombopoietin. Blood, 2001, vol. 98, 3241-3248
- **BAUTISTA, A. C** ; **SALIMI-MOOSAVI, H.** ; **JAWA, V.** Universal immunoassay applied during early development of large molecules to understand impact of immunogenicity on biotherapeutic exposure.. AAPS J, 2012, vol. 14, 843-849
- **SAUERBORN, M.** ; **BRINKS, V.** ; **JISKOOT, W.** ; **SCHELLEKENS, H.** Immunological mechanism underlying the immune response to recombinant human protein therapeutics. Trends Pharmacol Sci, 2010, vol. 31, 53-59
- **BAKER, M. P** ; **REYNOLDS, H. M** ; **LUMICISI, B.** ; **BRYSON, C. J.** Immunogenicity of protein therapeutics: The key causes, consequences and challenges. Self Nonself, 2010, vol. 1, 314-322

- **SAUNA, Z. E** ; **LAGASSÉ, D.** ; **PEDRAS-VASCON-CELOS, J.** ; **GOLDING, B.** ; **ROSENBERG, A. S**. Evaluating and Mitigating the Immunogenicity of Therapeutic Proteins.. *Trends Biotechnol*, 2018, vol. 36, 1068-1084
- **MANTEL, N.** Evaluation of survival data and two new rank order statistics arising in its consideration.. *Cancer Chemother Rep*, 1966, vol. 50, 163-170
- **PETO, R.** ; **PETO, J.** Asymptotically Efficient Rank Invariant Test Procedures. *Journal of the Royal Statistical Society. Series A (General)*, 1972, vol. 135, 185-207
- **SAUERBORN, M.** ; **BRINKS, V** ; **JISKOOT, W.** ; **SCHELLEKENS, H**. Immunological mechanism underlying the immune response to recombinant human protein therapeutics. *Trends Pharmacol Sci*, 2010, vol. 31, 53-59
- **BAKER, M. P** ; **REYNOLDS, H. M** ; **LUMICISI, B.** ; **BRYSON, C. J**. Immunogenicity of protein therapeutics. The key causes consequences and challenges. *Self Nonself*, 2010, vol. 1, 314-322
- **WURTH C.** ; **DEMEULE B.** ; **MAHLER H.-C.** ; **ADLER M.** Quality by Design Approaches to Formulation Robustness - An Antibody Case Study J.. *Pharma. Sci.*, 2016, vol. 105 (5), 1667-1675